# EUROPEAN PATENT APPLICATION

(11) **EP 4 272 758 A1**
(43) Date of publication of application: **08.11.2023**
(21) Application number: 21898094.4
(22) Date of filing: 26.11.2021
(51) Int. Cl.: A61K 45/00, A61K 31/47, A61K 31/4709, A61P 25/02, A61P 29/02, C07K 14/705, C12N 15/12

(54) **MEDICINE FOR ALLEVIATING NEUROPATHIC PAIN**

(30) Priority: 30.11.2020 JP 2020199206
(71) Applicant: Asahi Kasei Pharma Corporation, Chiyoda-ku Tokyo 100-0006 (JP)
(72) Inventor: IMAI, Yosuke, Tokyo 100-0006 (JP); YAMAMOTO, Hikaru, Tokyo 100-0006 (JP)
(74) Representative: Ingham, Stephen Howard
(86) International application number: PCT/JP2021/043375
(87) International publication number: WO 2022/114122

(57) **Abstract**

The present invention provides compounds that attenuate the function of the P2X7 receptor for the treatment of human neuropathic pain.

## Description

### [Technical Field]

The present invention relates to a medicament or the like for alleviating neuropathic pain.

### [Background Art]

There are various methods for classifying pain. One of them is based on the cause, and is classified into nociceptive pain, neuropathic pain, and psychogenic pain. Other classifications include, for example, chronic pain and acute pain. Neuropathic pain is a general term for pain caused by neuropathy.

Neuropathic pain is generally defined as "pain caused by a lesion or disease of the somatosensory nervous system". It is known to occur when a lesion or disease is present in any of the nociceptive signaling pathways from the peripheral nerves to the cerebrum (Non-Patent Literature 1).

Neuropathic pain is classified into two broad categories, peripheral neuropathic pain and central neuropathic pain depending on the site of nerve injury, and then known causes thereof include nutritional metabolic diseases, traumatic diseases, ischemic diseases, addictive diseases, hereditary diseases, infectious diseases, compression/strangulation diseases, immune diseases, neoplastic diseases, degenerative diseases and the like.

Known neuropathic pain includes postherpetic neuralgia (PHN), post-traumatic peripheral neuropathic pain, diabetic peripheral neuropathic pain (DPNP), trigeminal neuralgia (TGN), neuropathic pain after spinal code industry, and chemotherapy-induced peripheral neuropathic pain (CIPNP; or also called CIPN) (Non-Patent Literature 1).

Diabetic peripheral neuropathy (DPN) is a general term for various peripheral neuropathy found in diabetic patients, and is a peripheral neuropathy caused by insufficient insulin action or chronic hyperglycemic condition. Diabetic peripheral neuropathy is thought to develop as two major factors, peripheral nerve metabolism disorder and angiopathy caused by hyperglycemia, and then various hypotheses for the development including the accumulation of intraneuronal sorbitol due to increased polyol metabolism, protein glycation, free radicals, abnormalities in neurotrophic factors, and neuroischemia due to microangiopathy are proposed (Non-Patent Literature 2).

Pregabalin and gabapentin are known as drugs for neuropathic pain (Non-Patent Literature 3). The binding of these drugs to the voltage-gated calcium channel α2δ subunit causes a decrease in central sensitization or nociceptive transmission (Non-Patent Literature 3).

Further, tricyclic antidepressants (TCA) such as amitriptyline, nortriptyline, and desipramine are known as a drug for neuropathic pain (Non-Patent Literature 3).

In addition, serotonin norepinephrine reuptake inhibitors (SNRIs), such as duloxetine and venlafaxine, may also be a drug therapy option in neuropathic pain (Non-Patent Literature 3).

In addition, capsaicin and opioids such as tramadol and tapentadol are also known to be useful in neuropathic pain (Non-Patent Literature 3).

On the other hand, ATP receptors are classified into two broad categories, ion channel-embedded receptors (P2Xs) and G protein-coupled receptors (P2Ys), and then 7 subtypes (P2X1 to P2X7) and 8 subtypes (P2Y1, P2Y2, P2Y4, P2Y6, P2Y11 to P2Y14) have been reported, respectively (Non-Patent Literature 4).

A P2X7 receptor is expressed in microglia, etc. which is present in the posterior horn of the spinal cord and its involvement with neuropathic pain has been reported. For example, it has been reported that neuropathic hypersensitivity to mechanical and thermal stimuli completely disappeared in studies of the nerve ligation model using P2X7 receptor-deficient mice (Non-Patent Literature 5).

It has also been suggested that a P2X7 receptor is able to be a target for pain (Non-Patent Literature 6). Specific documents reported on P2X7 receptor antagonists are as follows ((1) to (4) below).

(1) It has been reported that a P2X7 receptor antagonist, which is a pyroglutamic acid amide derivative, showed an excellent activity in an animal model (Non-Patent Literature 7) .
(2) It has been reported that a P2X7 receptor antagonist, which is A438079 hydrochloride, suppressed the development of hypersensitivity to mechanical stimulation (Non-Patent Literature 8).
(3) It has been reported that N-(1-{ [(cyanoimo) (5-quinolinylamino)methyl]amino}-2,2-dimethylpropyl)-2-(3,4-dimethoxyphenyl)acetamide (A-740003; a selective P2X7 receptor antagonist) reduced neuropathic pain in rats in a dose-dependent manner (Non-Patent Literature 9). In the same document, it is also reported that A-740003 showed 40 nM (in human-derived cells) and 18 nM (in rat-derived cells) as IC₅₀ in the intracellular calcium measurement under stimulation by an agonist.
(4) It has been reported that JNJ-47965567 (30 mg/kg), a selective human P2X7 receptor antagonist, alleviated the amphetamine-induced hypersensitivity and showed not a large but a significant efficacy in neuropathy model rats. (Non-Patent Literature 10).

In addition, it has been reported that a pharmaceutical composition having a P2X7 receptor inhibitory activity and useful for treating pain and the like (Patent Literature 1), a novel compound having a P2X7 receptor inhibitory activity (Patent Literature 2), and a heterocyclic P2X7 antagonist (Patent Literature 3), P2X7 receptor antagonists and agonists (Patent Literature 4), and the use of tetrahydroquinoline derivatives in the treatment of various disorders mediated by P2X7 receptors (Patent Literature 5)

On the other hand, it has been also reported that P2X7 receptor knockout mice were susceptible to bone cancer pain and that the P2X7 receptor antagonist A-438079 could not alleviate bone cancer-related behavior in animal models of bone cancer. (Non-Patent Literature 6).

In addition, it has been reported that mutations in the human P2X7 receptor gene encoding the P2X7 receptor cause changes the function and expression of the P2X7 receptor, such as receptor transport, ATP binding, channel function and pore structure, which leads to a loss-of-function type (LOF type) phenotype and a gain-of-function type (GOF type) phenotype, and further it is pointed out that mutations in the P2X7 receptor gene may be involved in pain sensitivity in humans (Non-Patent Literature 11).

Specifically, many single nucleotide polymorphisms (SNPs) have been reported for the P2X7 receptor gene (Non-Patent Literatures 13 to 16). Examples of typical single nucleotide polymorphisms include the following (1) to (4).

### (1) rs208294 (H155Y)

It is a single nucleotide polymorphism in a GOF type (Non-Patent Literature 13). The polymorphism can occur when the 489th "C" of the P2X7 receptor gene is mutated to "T" (Non-Patent Literature 14). It has been reported that the minor allele frequency (MAF) value is "0.439" (Non-Patent Literature 15).

### (2) rs7958311 (R270H)

It is a single nucleotide polymorphism in a LOF type (Non-Patent Literature 13). The polymorphism can occur when the 853rd "G" of the P2X7 receptor gene is mutated to "A" (Non-Patent Literature 14). It has been reported that the minor allele frequency (MAF) value is "0.255" (Non-Patent Literature 15).

### (3) rs1718119 (A348T)

It is a single nucleotide polymorphism in a GOF type (Non-Patent Literature 13). The polymorphism can occur when the 1068th "G" of the P2X7 receptor gene is mutated to "A" (Non-Patent Literature 14). It has been reported that the minor allele frequency (MAF) value is "0.400" (Non-Patent Literature 15).

### (4) rs3751143 (E496A)

It is a single nucleotide polymorphism in a loss-of-function type (Non-Patent Literature 13). The polymorphism can occur by mutating the 1513th "A" of the P2X7 receptor gene to "C" (Non-Patent Literature 14). It has been reported that the minor allele frequency (MAF) value is "0.175" (Non-Patent Literature 15).

Furthermore, it has been reported that the presence of H155Y and A348T (both are single nucleotide polymorphisms in a gain-of-function type) correlates with strong pain in diabetic peripheral neuropathic pain (DPNP) in female patients. (Patent Literature 13).

### [Citation List]

### [Patent Literature]

[Patent Literature 1] JP 2019-182865 A
[Patent Literature 2] JP 2019-182806 A
[Patent Literature 3] JP 2020-518555 A
[Patent Literature 4] JP 2018-162269 A
[Patent Literature 5] JP 2020-514371 A
[Patent Literature 6] WO 2014-192698

### [Non-Patent Literature]

[Non-Patent Literature 1] Guidelines for the Pharmacologic Management of Neuropathic Pain, 2nd Edition (July 30, 2016, the 2nd impression of the 2nd edition)
[Non-Patent Literature 2] Diabetic neuropathy -From basic to clinical (Japanese book, March 28, 2013, the first impression of the first edition)
[Non-Patent Literature 3] Treatments for neuropathic pain, Clinical Pharmacist (2017), Vol. 9, No. 12
[Non-Patent Literature 4] The Journal of Biochemistry, (2009), Vol. 81, No. 10, pp. 884-890
[Non-Patent Literature 5] Pain (2005), Vol. 114, No. 3, pp. 386-396
[Non-Patent Literature 6] Pain (2011), Vol. 152, No. 8, pp. 1766-1776
[Non-Patent Literature 7] Bioorganic & Medicinal Chemistry Letters 2010, Vol. 20, pp. 5080-5084
[Non-Patent Literature 8] Neuroscience Letters 2011, Vol. 504, pp. 57-61
[Non-Patent Literature 9] The Journal of Pharmacology and Experimental Therapeutics 2006, Vol. 319, pp. 1376-1385
[Non-Patent Literature 10] Br J Pharmacol. 2013, Vol. 170, pp. 624-640
[Non-Patent Literature 11] Pain, 2018, Vol. 159, pp. 1064-1073
[Non-Patent Literature 12] Frontiers in Pharmacology, 2018, Vol. 9, Article 52, pp. 1-31
[Non-Patent Literature 13] Molecular Pain 2014, 10:37, pp. 1-11
[Non-Patent Literature 14] Purinergic Signal, 2009, Vol. 5, No. 2, pp. 257-262
[Non-Patent Literature 15] The FASEB Journal 2010, Vo. 24, pp. 2916-2927
[Non-Patent Literature 16] Nat Med 2013, Vol. 18, pp. 595-599
[Non-Patent Literature 17] Diabetic neuropathy -From basic to clinical 2013, pp. 2-4, (Japanese book, the first impression of the first edition, Nakayama Shoten Co., Ltd.)
[Non-Patent Literature 18] Guidelines for clinical assessment of neuropathic pain therapeutic agents (draft), 2020, Pharmaceutical Safety and Environmental Health Bureau, Ministry of Health, Labor and Welfare, Japan
[Non-Patent Literature 19] The Journal of Japan Society for Clinical Anesthesia, 2009, Vol. 29, No. 1, pp. 35-42
[Non-Patent Literature 20] Clinical Neurology, 2009 Vol. 49, No. 4, pp. 149-157
[Non-Patent Literature 21] Pharmacia, 2012, Vol. 48, No. 8, pp. 761-766

### [Summary of Invention]

### [Problems to be solved by Invention]

An object of the present invention is to provide a medicament, etc. for alleviating neuropathic pain.

### [Means for Solving Problems]

One embodiment of the invention is characterized in that a medicament for alleviating (improving or treating) diabetic peripheral neuropathic pain (DPNP), etc., which comprises a compound having a P2X7 receptor inhibitory (or antagonist) activity for attenuating the function of the P2X7 receptor (including the human P2X7 receptor), wherein the compound may be a P2X7 receptor inhibitor (or antagonist), a P2X7 receptor inhibitory (or antagonist) agent, or a P2X7 receptor inhibitory (or antagonist) drug. Another embodiment of a medicament of the present invention is a medicament for alleviating neuropathic pain, which is administered to a patient in which the function of the P2X7 receptor is enhanced.

Namely, the present invention relates to the following inventions and the like.
[1] A medicament for alleviating (or treating) at least one neuropathic pain, particularly human diabetic peripheral pain, selected from diabetic peripheral neuropathic pain (DPNP) and human post surgical pain (PSP), wherein the medicament comprises a compound which attenuates the function of the P2X7 receptor as an active ingredient.
   [1-2] A medicament for alleviating (or treating) human diabetic peripheral neuropathic pain (DPNP), wherein the medicament comprises a compound which attenuates the function of the P2X7 receptor as an active ingredient.
   [1-3] A medicament for alleviating (or treating) human postoperative pain (PSP), wherein the medicament comprises a compound which attenuates the function of the P2X7 receptor as an active ingredient.
[2] The medicament according to any one of the above [1] to [1-3], wherein the P2X7 receptor is present in the central system (or central nervous system).
[3] The medicament according to any one of the above [1] to [2], wherein the compound which attenuates the function of the P2X7 receptor migrates to/reaches (or can migrate to/reach) the central system (or central nervous system).
   In addition, when the cited item numbers are indicated as a range like [1] to [2] as mentioned above, and an item indicated with an item number having a sub-number such as [1-2] is included in the range, it means that the item indicated with the item number having a sub-number such as [1-2] is also cited. This rule also holds for the following descriptions.
[4] The medicament according to any one of the above [1] to [3], wherein the compound that attenuates the function of the P2X7 receptor is at least one selected from the compounds described in Patent Literature 5 (JP 2020-514371 A), for example, any of the following compounds and salts thereof:
   (5S,8S)-N-(2,4-dichloro-6-fluorobenzyl)-5-fluoro-8-hydroxy-8-(hydroxymethyl)-5,6,7,8-tetrahydroquinoline-5-carboxamide (Compound 1);
   (5S,8S)-N-(2-chloro-4,6-difluorobenzyl)-5-fluoro-8-hydroxy-8-(hydroxymethyl)-5,6,7,8-tetrahydroquinoline-5-carboxamide (Compound 2);
   (5S,8S)-N-(2,4-dichloro-6-(hydroxymethyl)benzyl)-5-fluoro-8-hydroxy-8-(hydroxymethyl)-5,6,7,8-tetrahydroquinoline-5-carboxamide (Compound 3);
   (5S,8S)-N-((R)-1-(2-chloro-4-fluorophenyl)ethyl)-5-fluoro-8-hydroxy-8-(hydroxymethyl)-5,6,7,8-tetrahydroquinoline-5-carboxamide (Compound 4);
   (5S,8S)-N-((3,5-dichloropyridin-2-yl)methyl)-5-fluoro-8-hydroxy-8-(hydroxymethyl)-5,6,7,8-tetrahydroquinoline-5-carboxamide (Compound 5)
   (5S,8S)-N-((R)-1-(2,4-dichlorophenyl)ethyl)-5-fluoro-8-hydroxy-8-(hydroxymethyl)-5,6,7,8-tetrahydroquinoline-5-carboxamide (Compound 6);
   (5S,8S)-N-(2-chloro-4-fluorobenzyl)-5-fluoro-8-hydroxy-8-(hydroxymethyl)-5,6,7,8-tetrahydroquinoline-5-carboxamide (Compound 7);
   (5S,8S)-N-(2-chloro-3,4-difluorobenzyl)-5-fluoro-8-hydroxy-8-(hydroxymethyl)-5,6,7,8-tetrahydroquinoline-5-carboxamide (Compound 8);
   (5S,8S)-N-(2,4-dichloro-3-fluorobenzyl)-5-fluoro-8-hydroxy-8-(hydroxymethyl)-5,6,7,8-tetrahydroquinoline-5-carboxamide (Compound 9); and
   (5S,8S)-N-(2,4-dichlorobenzyl)-5-fluoro-8-hydroxy-8-(hydroxymethyl)-5,6,7,8-tetrahydroquinoline-5-carboxamide (Compound 10).

   [4-2] The medicament according to any one of the above [1] to [3], wherein the compound that attenuates the function of the P2X7 receptor is (5S,8S)-N-(2,4-dichloro-6-fluorobenzyl)-5-fluoro-8-hydroxy-8-(hydroxymethyl)-5,6,7,8-tetrahydroquinoline-5-carboxamide (Compound 1) or a salt thereof.
   [4-3] The medicament according to any one of the above [1] to [3], wherein the compound that attenuates the function of the P2X7 receptor is (5S,8S)-N-(2-chloro-4,6-difluorobenzyl)-5-fluoro-8-hydroxy-8-(hydroxymethyl)-5,6,7,8-tetrahydroquinoline-5-carboxamide (Compound 2) or a salt thereof.
   [4-4] The medicament according to any one of the above [1] to [3], wherein the compound that attenuates the function of the P2X7 receptor is (5S,8S)-N-(2,4-dichloro-6-(hydroxymethyl)benzyl)-5-fluoro-8-hydroxy-8-(hydroxymethyl)-5,6,7,8-tetrahydroquinoline-5-carboxamide (Compound 3) or a salt thereof.
   [4-5] The medicament according to any one of the above [1] to [3], wherein the compound that attenuates the function of the P2X7 receptor is (5S,8S)-N-((R)-1-(2-chloro-4-fluorophenyl)ethyl)-5-fluoro-8-hydroxy-8-(hydroxymethyl)-5,6,7,8-tetrahydroquinoline-5-carboxamide (Compound 4) or a salt thereof.
   [4-6] The medicament according to any one of the above [1] to [3], wherein the compound that attenuates the function of the P2X7 receptor is (5S,8S)-N-((3,5-dichloropyridin-2-yl)methyl)-5-fluoro-8-hydroxy-8-(hydroxymethyl)-5,6,7,8-tetrahydroquinoline-5-carboxamide (Compound 5) or a salt thereof.
   [4-7] The medicament according to any one of the above [1] to [3], wherein the compound that attenuates the function of the P2X7 receptor is (5S,8S)-N-((R)-1-(2,4-dichlorophenyl)ethyl)-5-fluoro-8-hydroxy-8-(hydroxymethyl)-5,6,7,8-tetrahydroquinoline-5-carboxamide (Compound 6) or a salt thereof.
   [4-8] The medicament according to any one of the above [1] to [3], wherein the compound that attenuates the function of the P2X7 receptor is (5S,8S)-N-(2-chloro-4-fluorobenzyl)-5-fluoro-8-hydroxy-8-(hydroxymethyl)-5,6,7,8-tetrahydroquinoline-5-carboxamide (Compound 7) or a salt thereof.
   [4-9] The medicament according to any one of the above [1] to [3], wherein the compound that attenuates the function of the P2X7 receptor is (5S,8S)-N-(2-chloro-3,4-difluorobenzyl)-5-fluoro-8-hydroxy-8-(hydroxymethyl)-5, 6,7,8-tetrahydroquinoline-5-carboxamide (Compound 8) or a salt thereof.
   [4-10] The medicament according to any one of the above [1] to [3], wherein the compound that attenuates the function of the P2X7 receptor is (5S,8S)-N-(2,4-dichloro-3-fluorobenzyl)-5-fluoro-8-hydroxy-8-(hydroxymethyl)-5,6,7,8-tetrahydroquinoline-5-carboxamide (Compound 9) or a salt thereof.
   [4-11] The medicament according to any one of the above [1] to [3], wherein the compound that attenuates the function of the P2X7 receptor is (5S,8S)-N-(2,4-dichlorobenzyl)-5-fluoro-8-hydroxy-8-(hydroxymethyl)-5,6,7,8-tetrahydroquinoline-5-carboxamide (Compound 10) or a salt thereof.
[5] The medicament according to any one of the above [1] to [4-11], wherein the medicament is for administering to a patient with the P2X7 receptor gain-of-function type or a patient with the P2X7 receptor unchanged function type (particularly, a patient with the P2X7 receptor gain-of-function type).
[6] A method for alleviating or treating at least one neuropathic pain selected from diabetic peripheral neuropathic pain (DPNP) and postoperative pain (PSP), wherein the method comprises administering a compound [or a medicament comprising the compound (as an active ingredient)] that attenuates the function of the P2X7 receptor to humans [or patients / humans or patients with at least one neuropathic pain selected from diabetic peripheral neuropathic pain (DPNP) and postoperative pain (PSP), (particularly, human diabetic peripheral neuropathic pain).
   [6-2] A method for alleviating or treating diabetic peripheral neuropathic pain (DPNP), wherein the method comprises administering the compound that attenuates the function of the P2X7 receptor [or a medicament comprising the compound (as an active ingredient)] to humans [or patients / humans or patients with diabetic peripheral neuropathic pain (DPNP)].
   [6-3] A method for alleviating or treating postoperative pain (PSP), wherein the method comprises administering a compound [or a medicament comprising the compound (as an active ingredient)] that attenuates the function of the P2X7 receptor to humans [or patients / humans or patients with postoperative pain (PSP)].
[7] The method according to any one of the above [6] to [6-3], wherein the P2X7 receptor is present in the central system (or central nervous system).
[8] The method according to any one of the above [6] to [7], wherein the compound which attenuates the function of the P2X7 receptor migrates to/reaches (or can migrate to/reach) the central system (or central nervous system).
[9] The method according to any one of the above [6] to [8], wherein the compound that attenuates the function of the P2X7 receptor is at least one selected from the compounds described in Patent Literature 5 (JP 2020-514371 A), for example, any of the following compounds and salts thereof:
   (5S,8S)-N-(2,4-dichloro-6-fluorobenzyl)-5-fluoro-8-hydroxy-8-(hydroxymethyl)-5,6,7,8-tetrahydroquinoline-5-carboxamide (Compound 1);
   (5S,8S)-N-(2-chloro-4,6-difluorobenzyl)-5-fluoro-8-hydroxy-8-(hydroxymethyl)-5,6,7,8-tetrahydroquinoline-5-carboxamide (Compound 2);
   (5S,8S)-N-(2,4-dichloro-6-(hydroxymethyl)benzyl)-5-fluoro-8-hydroxy-8-(hydroxymethyl)-5,6,7,8-tetrahydroquinoline-5-carboxamide (Compound 3);
   (5S,8S)-N-((R)-1-(2-chloro-4-fluorophenyl)ethyl)-5-fluoro-8-hydroxy-8-(hydroxymethyl)-5,6,7,8-tetrahydroquinoline-5-carboxamide (Compound 4);
   (5S,8S)-N-((3,5-dichloropyridin-2-yl)methyl)-5-fluoro-8-hydroxy-8-(hydroxymethyl)-5,6,7,8-tetrahydroquinoline-5-carboxamide (Compound 5)
   (5S,8S)-N-((R)-1-(2,4-dichlorophenyl)ethyl)-5-fluoro-8-hydroxy-8-(hydroxymethyl)-5,6,7,8-tetrahydroquinoline-5-carboxamide (Compound 6);
   (5S,8S)-N-(2-chloro-4-fluorobenzyl)-5-fluoro-8-hydroxy-8-(hydroxymethyl)-5,6,7,8-tetrahydroquinoline-5-carboxamide (Compound 7);
   (5S,8S)-N-(2-chloro-3,4-difluorobenzyl)-5-fluoro-8-hydroxy-8-(hydroxymethyl)-5,6,7,8-tetrahydroquinoline-5-carboxamide (Compound 8);
   (5S,8S)-N-(2,4-dichloro-3-fluorobenzyl)-5-fluoro-8-hydroxy-8-(hydroxymethyl)-5,6,7,8-tetrahydroquinoline-5-carboxamide (Compound 9); and
   (5S,8S)-N-(2,4-dichlorobenzyl)-5-fluoro-8-hydroxy-8-(hydroxymethyl)-5,6,7,8-tetrahydroquinoline-5-carboxamide (Compound 10).
[10] The method according to any one of the above [6] to [9], wherein the patient is a patient with the P2X7 receptor gain-of-function type or a patient with the P2X7 receptor unchanged function type, (particularly, a patient with the P2X7 receptor gain-of-function type).
[11] The method according to any one of the above [6] to [10], wherein the compound which attenuates the function of the P2X7 receptor migrates to/reaches the central system (or central nervous system).
[12] A medicament for alleviating human neuropathic pain, which is administered to a patient with the P2X7 receptor gain-of-function type.
[13] The medicament according to the above [12], wherein said human neuropathic pain is human diabetic peripheral neuropathic pain (DPNP).
[14] The medicament according to the above [12] or [13], wherein the medicament comprises a compound which attenuates the function of the P2X7 receptor and at least one compound (or medicament) selected from a different type from the said compound (or medicament) for alleviating neuropathic pain.
[15] A method for alleviating or threating human neuropathic pain, wherein the method comprises administering a medicament [or a compound/drug (therapeutic drug) for human neuropathic pain] for alleviating human neuropathic pain to a patient with the P2X7 receptor GOF type.
[16] The method according to the above [15], wherein said human neuropathic pain is human diabetic peripheral neuropathic pain (DPNP).
[17] The method according to the above [15] or [16], wherein the medicament for alleviating human neuropathic pain comprises a compound which attenuates the function of the P2X7 receptor and at least one compound (or medicament) selected from a different type from the compound (or medicament) for alleviating neuropathic pain.

### [Effects of Invention]

According to the present invention, a medicament for alleviating human neuropathic pain, a method for alleviating or treating human neuropathic pain, and the like are provided.

### [Brief Description of Drawings]

[FIG. 1] Figure 1 is a diagram for explaining the classification (type) of pain scores in Example 5.

### [Description of Embodiments]

Hereinafter, the present invention shall be described in detail on the basis of specific embodiments. However, the present invention is not intended to be confined to the following embodiments, and can be carried out in any embodiments within the range that would not depart from the spirit of the present invention.

### 1. Embodiment 1

One embodiment of the medicament of the present invention is a medicament for alleviating diabetic peripheral neuropathic pain (DPNP), etc., which comprises a compound that attenuates the function of the P2X7 receptor as an active ingredient.

One embodiment of the therapeutic method of the present invention is a method for treating diabetic peripheral neuropathic pain, which comprises administering a compound that attenuates the function of the P2X7 receptor to a patient with a diabetic peripheral neuropathic pain (DPNP), etc.

One embodiment of the alleviating method of the present invention is a method for alleviating diabetic peripheral neuropathic pain, which comprises administering a compound that attenuates the function of the P2X7 receptor to a patient with a diabetic peripheral neuropathic pain (DPNP), etc.

In one embodiment, the present invention also provides the use of a compound that attenuates the function of the P2X7 receptor for the manufacture of a medicament for treating neuropathic pain in a patient.

In one embodiment, the present invention also provides the use of a compound that attenuates the function of the P2X7 receptor for the manufacture of a medicament for treating diabetic peripheral neuropathic pain in a patient.

In one embodiment, the present invention also provides the use of a compound that attenuates the function of the P2X7 receptor for the manufacture of a medicament for treating postoperative pain in a patient.

In one embodiment, the present invention also provides the use of a compound that attenuates the function of the P2X7 receptor for the manufacture of a medicament for treating diabetic peripheral neuropathic pain in a patient wherein the patient is a patient with the P2X7 receptor gain-of-function type or a patient with the P2X7 receptor unchanged function type.

In one embodiment, the present invention also provides the use of a compound that attenuates the function of the P2X7 receptor for the manufacture of a medicament for treating diabetic peripheral neuropathic pain in a patient wherein the patient is a patient with the P2X7 receptor gain-of-function type.

In one embodiment, the present invention also provides the use of a compound that attenuates the function of the P2X7 receptor for the manufacture of a medicament for treating postoperative pain in a patient wherein the patient is a patient with the P2X7 receptor gain-of-function type or a patient with the P2X7 receptor unchanged function type.

In one embodiment, the present invention also provides the use of a compound that attenuates the function of the P2X7 receptor for the manufacture of a medicament for treating postoperative pain in a patient wherein the patient is a patient with the P2X7 receptor gain-of-function type.

In one embodiment, the present invention also provides the use of a compound that attenuates the function of the P2X7 receptor for the manufacture of a medicament for treating diabetic peripheral neuropathic pain in a patient wherein the patient is a patient with the P2X7 receptor gain-of-function type and wherein the compound that attenuates the function of the P2X7 receptor is(5S,8S)-N-(2-chloro-4-fluorobenzyl)-5-fluoro-8-hydroxy-8-(hydroxymethyl)-5,6,7,8-tetrahydroquinoline-5-carboxamide, or pharmaceutically acceptable salt thereof.

### (1) P2X7 receptor

In the present invention, the P2X7 receptor (P2RX7) belongs to the family of P2X ionotropic receptors that are activated by extracellular nucleotides, (e.g., adenosine triphosphate (ATP)), and is not particularly limited as long as it is a non-selective cation channel that requires the high concentrations (specifically about 0.1 to 1 mM range) of extracellular nucleotides for its activate (Patent Literature 5 and Non-Patent Literature 4).

The origin of the P2X7 receptor according to the present invention is not particularly limited, and may be, for example, mouse or human, but a human-derived P2X7 receptor is preferably used. The human-derived P2X7 receptor includes, for example, GenBank accession number Y09561. Specifically, the P2X7 receptor according to the present invention includes the P2X7 receptor described in SEQ ID NO: 2 in the sequence list, and preferable examples of the gene encoding the P2X7 receptor according to the present invention include the P2X7 receptor gene shown in SEQ ID NO: 1 in the sequence list.

Many single nucleotide polymorphisms have been observed in the P2X7 receptor gene encoding the P2X7 receptor (Non-Patent Literatures 13 to 16). The P2X7 receptor according to the present invention includes not only the wild-type P2X7 receptor gene but also the P2X7 receptor encoded by the mutant P2X7 receptor gene observed in nature.

The tissues or cells in which the P2X7 receptor according to the present invention is expressed in a living body include, but not particularly limited to for example, macrophages and monocytes in the periphery, and those present in glial cells such as microglia and astrocytes in the central nervous system (CNS) (Patent Literature 5, Non-Patent Literature 11). In general, pain follows a path: nociceptors (primary neurons of nerve cells) → dorsal horn of spinal cord (secondary neurons) → thorax (tertiary neurons) → cerebral cortex. Therefore, the P2X7 receptor according to the present invention present in the central system (or central nervous system, *e.g.,* brain and spinal cord), such as the P2X7 receptor in glial cells present in or near the dorsal horn of the spinal cord is preferable.

### (2) Active ingredient (compound that attenuates the function of P2X7 receptor)

As described above, the P2X7 receptor is expressed in microglia, etc. which is present in the posterior horn of the spinal cord, and its involvement with neuropathic pain has been reported. For example, it has been reported that neuropathic hypersensitivity to mechanical and thermal stimuli completely disappeared in studies of the nerve ligation model using P2X7 receptor-deficient mice (Non-Patent Literature 5). It has also been suggested that the P2X7 receptor is able to be a target for pain (Non-Patent Literature 6).

Therefore, the compound which attenuates the function of the P2X7 receptor may be the compound that migrates to/reaches (or can migrate to/reach) the central system (or central nervous system, *e.g.*, brain and spinal cord).

Furthermore, patients with diabetic peripheral neuropathic pain (DPNP) according to the present invention include a patient with the P2X7 receptor gene gain-of-function type (GOF type) haplotype on both chromosomes (hereinafter, it may be referred to as a GOF type patient with a diabetic peripheral neuropathic pain) and a patient with the P2X7 receptor gene of a loss-of-function type (LOF type) haplotype on one or both chromosomes (wherein when only one chromosome shows LOF haplotype, the other chromosome is standard haplotype; hereinafter, it may be referred to as a LOF type patient with diabetic peripheral neuropathic pain). As a result of comparing the GOF type patient with diabetic peripheral neuropathic pain with the LOF type patient with diabetic peripheral neuropathic pain in terms of the degree of pain, it has been surprisingly found that pain in the LOF type patient with diabetic peripheral neuropathic pain is significantly lower than pain in the GOF type patient with diabetic peripheral neuropathic pain (Example 5 of the present application).

Based on the above, the active ingredient in the present invention is a compound that "attenuates the function of the P2X7 receptor" and is not particularly limited as long as its application achieves alleviating neuropathic pain.

In general, it is known that the P2X7 receptor is activated by a sustained high concentration of extracellular ATP to form a non-selective pore structure that promotes the influx of substances of about 900 Da and cations such as sodium ion, calcium ion, and potassium ion (Non-Patent Literature 12). Therefore, suppressing or preventing such activation by ATP and subsequent formation of a non-selective pore structure is also considered as one embodiment of "attenuating the function of the P2X7 receptor".

In addition, the P2X7 receptor is expressed on the surface of several cell types, especially on the surface of cell types known to be involved in inflammatory and immune processes (Patent Literature 5). Since, for example, activation of the P2X7 receptor by extracellular ATP leads to the release of IL-1β and IL-18, giant cell formation, degranulation, and L-selectin shedding (Patent Literature 5), suppressing or preventing at least a part of these series of reactions is also considered as one embodiment of "attenuating the function of the P2X7 receptor".

On the other hand, it has also been reported that pain behavior such as allodynia is suppressed by antisense oligos and siRNA against receptors such as P2X3 belonging to the P2X ion channel type receptor family as well as P2X7 receptors (Non-Patent Literature 4). Therefore, suppressing or preventing the expression of the P2X7 receptor is also considered as one embodiment of "attenuating the function of the P2X7 receptor".

Here, the compound acting as an antagonist on the P2X7 receptor is able to suppress or prevent the activation of the P2X7 receptor by high concentration of ATP and the subsequent cascade reaction, and thus can be preferably used as a compound that attenuates the function of the P2X7 receptor (Non-Patent Literatures 7 to 10 and Patent Literatures 1 to 5) .

The compound that attenuates the function of the P2X7 receptor may be selective for the P2X7 receptor [*i.e.*, a selective P2X7 receptor inhibitory (or antagonist) agent, or a selective P2X7 receptor inhibitory (antagonist) drug)].

Here, the molecular species of the "compound that attenuates the function of the P2X7 receptor" is not particularly limited, and examples include a low molecular weight compound, a peptide, an antibody and the like.

The P2X7 receptor is preferably a compound having high tissue infiltration since its expression is observed in peripheral tissues and the central nervous system (Patent Literature 5 and Non-Patent Literature 11). In particular, when acting "the compound that attenuates the function of the P2X7 receptor" to the P2X7 receptor present in the central nervous system, it is preferably a compound that crosses the blood-brain barrier, which is a barrier for further distribution from circulating blood to the central nervous system.

Examples include a low molecular weight compound as a compound that attenuates the function of the P2X7 receptor present in the peripheral nervous system and the central nervous system.

Examples of the low molecular weight compound include a compound having a molecular weight of about 800 or less. Examples of the upper limit of the molecular weight include preferably 600 or less, 500 or less, and 450 or less, and those of the lower limit of the molecular weight include, but not particularly limited to, 10 or more, 50 or more, and 100 or more. Examples include most preferably, a low molecular weight compound having a molecular weight of about 100 to 450. In general, a compound having a molecular weight of 450 or less is expected to pass passively through the blood-brain barrier (Non-Patent Literature 21). An appropriate lipophilicity with a cLogP value (common logarithmic value of octanol/water partition coefficient) of 3 or less of the compound as well as a low molecular weight of the compound becomes important for the permeation of the gastrointestinal membrane barrier and also leads optimization of passing the blood-brain barrier (Non-Patent Literature 21).

More specifically, preferable examples include 1) a pyroglutamic acid amide derivative (Non-Patent Literature 7), 2) a compound represented by the following formula (I) (Non-Patent Literature 8), 3) N-(1-{[(cyanoimino)(5-quinolinylamino)methyl]amino}-2,2-dimethylpropyl)-2-(3,4-dimetoxyphenyl)acetamide (Non-Patent Literature 9), and 4) a low molecular weight compound with a P2X7 receptor antagonist activity, such as a compound represented by the following formula (II) (JNJ 47965567; Non-Patent Literature 10) as "a low molecular weight compound that attenuates the function of the P2X7 receptor".

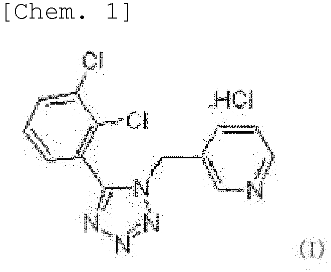

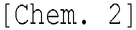

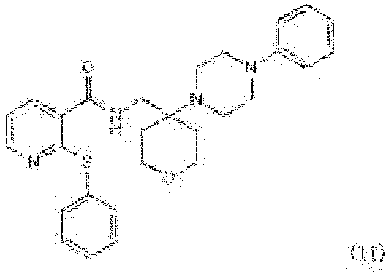

More preferable examples include the compounds described in Patent Literature 5 as a compound that attenuates the function of the P2X7 receptor because those compounds have a P2X7 receptor inhibitory activity *in vitro.* Concrete examples of the compound include the following compounds or salts thereof:
(5S,8S)-N-(2,4-dichloro-6-fluorobenzyl)-5-fluoro-8-hydroxy-8-(hydroxymethyl)-5,6,7,8-tetrahydroquinoline-5-carboxamide (Compound 1);
(5S,8S)-N-(2-chloro-4,6-difluorobenzyl)-5-fluoro-8-hydroxy-8-(hydroxymethyl)-5,6,7,8-tetrahydroquinoline-5-carboxamide (Compound 2);
(5S,8S)-N-(2,4-dichloro-6-(hydroxymethyl)benzyl)-5-fluoro-8-hydroxy-8-(hydroxymethyl)-5,6,7,8-tetrahydroquinoline-5-carboxamide (Compound 3);
(5S,8S)-N-((R)-1-(2-chloro-4-fluorophenyl)ethyl)-5-fluoro-8-hydroxy-8-(hydroxymethyl)-5,6,7,8-tetrahydroquinoline-5-carboxamide (Compound 4);
(5S,8S)-N-((3,5-dichloropyridin-2-yl)methyl)-5-fluoro-8-hydroxy-8-(hydroxymethyl)-5,6,7,8-tetrahydroquinoline-5-carboxamide (Compound 5)
(5S,8S)-N-((R)-1-(2,4-dichlorophenyl)ethyl)-5-fluoro-8-hydroxy-8-(hydroxymethyl)-5,6,7,8-tetrahydroquinoline-5-carboxamide (Compound 6);
(5S,8S)-N-(2-chloro-4-fluorobenzyl)-5-fluoro-8-hydroxy-8-(hydroxymethyl)-5,6,7,8-tetrahydroquinoline-5-carboxamide (Compound 7);
(5S,8S)-N-(2-chloro-3,4-difluorobenzyl)-5-fluoro-8-hydroxy-8-(hydroxymethyl)-5,6,7,8-tetrahydroquinoline-5-carboxamide (Compound 8);
(5S,8S)-N-(2,4-dichloro-3-fluorobenzyl)-5-fluoro-8-hydroxy-8-(hydroxymethyl)-5,6,7,8-tetrahydroquinoline-5-carboxamide (Compound 9); and
(5S,8S)-N-(2,4-dichlorobenzyl)-5-fluoro-8-hydroxy-8-(hydroxymethyl)-5,6,7,8-tetrahydroquinoline-5-carboxamide (Compound 10).

The salts of the compound are preferably a pharmaceutically acceptable salt. It would be appreciated that for use in medicament, the salts of the compounds of the present invention should be pharmaceutically acceptable. Suitable pharmaceutically acceptable salts would be apparent to a person skilled in the art and include those described in J. Pharm. Sci, 66, 1-19, 1977, such as acid addition salts formed with inorganic acids *e.g.,* hydrochloric acid, hydrobromic acid, sulfuric acid, nitric acid or phosphoric acid; and those formed with organic acids *e.g.,* succinic acid, maleic acid, formic acid, acetic acid, trifluoroacetic acid, propionic acid, fumaric acid, citric acid, tartaric acid, benzoic acid, p-toluenesulfonic acid, methanesulfonic acid or naphthalenesulfonic acid.

Among the compounds described in Patent Literature 5, the present inventors have found that Compound 7 or Compound 10 corresponds the low molecular weight compound that exhibits sufficient distribution to blood, brain and spinal cord (Examples 1 and 7). Therefore, the present inventors consider that Compound 7 or Compound 10, compounds similar to these compounds, and salts thereof (for example, compounds described in Patent Literature 5 such as the Compounds 1 to 6, 8, 9 and salts thereof) can sufficiently exhibit exposure to immune cells (e.g., macrophages) present in human blood and to tissues/cells (e.g., microglia, astrocytes) present in the central nervous system.

From the above, the most preferable examples include Compound 7 or Compound 10 or a salt thereof as a compound that attenuates the function of the P2X7 receptor. Further, the most preferable examples include Compound 7 as a compound that attenuates the function of the P2X7 receptor. Furthermore, the most preferable examples include Compound 10 as a compound that attenuates the function of the P2X7 receptor.

It should be noted that whether or not a certain compound attenuates the function of the P2X7 receptor can be confirmed by using a conventional *in vitro* assay method.

For example, in cells where the P2X7 receptor is stably expressed, the functional activity of the compound can be determined by measuring changes in intracellular calcium concentration using a Ca²⁺-sensitive fluorescent dye. It can be confirmed whether or not the compound attenuates the function of the human P2X7 receptor by assessing the effect of adding the compound to the assay system on the function (Patent Literature 5). Here, preferable examples include Fluo4 and Fura2 as the Ca²⁺ fluorescence indicator.

Alternatively, the assay system in which the nucleic acid fluorescence staining reagent YO-PRO-1 is incorporated into cells stably expressing the P2X7 receptor can be used together with or instead.

The means for activating the P2X7 receptor in the said cells is not particularly limited, and preferable examples include BzATP stimulation as a means.

### (3) Diabetic peripheral neuropathic pain, etc.

### (3-1) Neuropathic pain

Neuropathic pain is generally defined as "pain caused by a lesion or disease of the somatosensory nervous system" and is known to occur when a lesion or disease is present in any of the nociceptive signaling pathways from the peripheral nerves to the brain (Non-Patent Literature 1).

In addition, nociceptive pain is generally defined as "pain caused by excitement of nociceptive receptors due to substantial or potential damage to living tissues other than nerve tissue" (Non-Patent Literature 1).

Diseases associated with pain are classified into two broad categories, nociceptive pain and neuropathic pain, but both conditions are concepts that can be mixed because inflammation in the nervous tissue may excite nociceptive receptors to cause pain (Non-Pain Literature 1). In fact, post surgical pain (PSP), which is categorized as neuropathic pain, is understood to be pain that combines neuropathic and nociceptive components, and such mixed pain is also included in neuropathic pain according to the present invention.

Neuropathic pain is classified into two broad categories, peripheral neuropathic pain and central neuropathic pain depending on the site of nerve injury, and the known causes include nutritional metabolic diseases, traumatic diseases, ischemic diseases, addictive diseases, hereditary diseases, infectious diseases, compression/strangulation diseases, immune diseases, neoplastic diseases, and degenerative diseases. However, the neuropathic pain according to the present invention is not distinguished between peripheral and central, and the cause is not particularly limited. Preferable examples include peripheral neuropathic pain as neuropathic pain.

Examples includes postherpetic neuralgia (PHN), post-traumatic peripheral neuropathic pain, diabetic peripheral neuropathic pain (DPNP), trigeminal neuralgia (TGN), neuropathic pain after spinal code industry, chemotherapy-induced peripheral neuropathic pain (CIPNP; or also called CIPN), and post surgical pain (PSP) as neuropathic pain according to the present invention. Preferable examples of neuropathic pain include DPNP or PSP.

Shingles is a viral disease that generally develops mainly in adults, and is known as a disease that causes pain and rash (erythema and blister) along the running of peripheral nerves by activation of the varicella-zoster (HZ) virus lurking in the ganglia when the body's immunity weakens for some reason. The main symptoms of shingles are pain and rash. Pain is found along the nerve run caused by the HZ virus and is described as intolerable pain, stinging pain, burning pain, needlestick pain, tightening pain, and the like. Pain may continue for months to years or more even after the eruption disappears, and this neuralgia is thought to occur as a result of degeneration of nerves that have been severely damaged by the HZ virus and is referred to as postherpetic neuralgia (PHN).

### (3-2) Diagnosis of neuropathic pain

Existing guidelines for the assessment/diagnosis of neuropathic pain include the guidelines of the European Federation of Neurological Societies (EFNS) and the International Association for the Study of Pain (IASP), and the diagnostic algorithm (Grading system) created by the IASP's Neuropathic Pain Special Interest Group (NeuPSIG) is recommended (Non-Pain Literature 1). A method for assessing/diagnosing neuropathic pain based on the same diagnostic algorithm has been proposed regardless of a lesion or disease that causes neuropathic pain, and is widely used as the current international standard for diagnosing neuropathic pain (Non-Patent Literature 1).

Tests for assessing neurological lesions or disorders that explain neuropathic pain include imaging tests (MRI, CT), neurophysiological tests (Nerve conduction study, trigeminal nerve reflexes, laser-induced potentials (LEPs), corneal confocal microscopy (CCM), skin biopsy, and the like (Non-Patent Literature 1).

In general, neuropathic pain causes characteristic pain different from nociceptive pain. It includes spontaneous pain (persistent or intermittent) or stimulus-induced pain (allodynia and hyperalgesia) located in the area consistent with the damaged innervation area, and is characterized by a mixture of various sensory abnormalities caused by nerve damage (Non-Patent Literature 1).

Various screening tools (ID Pain, NPQ, pain DETECT, LANSS, DN4) are known as an aid to diagnosis (Non-Patent Literature 1).

### (3-3) Assessment of the intensity of neuropathic pain

In the present invention, the method for assessing the intensity of neuropathic pain is not particularly limited, and preferable examples include VAS (Visual Analog Scale) and NRS (Numeric Racing Scale) (Non-Patent Literatures 18 to 19).

In general, in the NRS, pain is classified into 11 points from 0 to 10, and the patient is asked the score of pain, wherein 0 is no pain and 10 is the worst pain imaginable, and in the VAS, the patient is asked to mark the place indicating the degree of pain, wherein the left end of the 100 mm line is "no pain" and the right end is "the worst pain". The assessment may be based on the subjective view of such a patient, and may be, for example, a method using a device for quantifying or analyzing perception or pain sensation (e.g., Pain Vision PS-2100; manufactured by Nipro) (Non-Patent Literature 19).

Since pain is subjective, it is standard practice to assess the patient's self-reported pain, but a healthcare professional may assess the patient's pain intensity on behalf of the patient (STAS-J). In addition, VAS and NRS are preferably used in patients with mild cognitive decline with an MMSE of about 18 points or more, and NRS is preferably used in patients with moderate cognitive decline with an MMSE of 10 to 17 points (2. Comprehensive assessment of pain, Guidelines for drug therapy for cancer pain, 2010 edition).

### (3-4) Diabetic peripheral neuropathic pain

Diabetic peripheral neuropathy (DPN) is a general term for various peripheral neuropathy found in diabetic patients, and is a peripheral neuropathy caused by insufficient insulin action or chronic hyperglycemic condition. Diabetic peripheral neuropathy is thought to develop as two major factors, peripheral nerve metabolism disorder and angiopathy caused by hyperglycemia, and then various hypotheses for the development including the accumulation of intraneuronal sorbitol due to increased polyol metabolism, protein glycation, free radicals, abnormalities in neurotrophic factors, and neuroischemia due to microangiopathy are proposed (Non-Patent Literature 2).

In addition, diabetic peripheral neuropathy (DPN) is often clinically classified by the Thomas classification. Here, it is classified into four types: "hyperglycemic neuropathy", "symmetric polyneuropathy", "local and multifocal neuropathy", and "mixed type" (Non-Patent Literature 17).

Hyperglycemic neuropathy refers to a condition characterized by a feeling of needle stick in the foot, which is seen in patients with persistent hyperglycemic conditions. Since this numbness or pain is alleviated by improving the glycemic control, hyperglycemic neuropathy is considered to be a physiological or functional symptom against the background of changes in channel function due to hyperglycemia. Therefore, hyperglycemic neuropathy may be more appropriate to call a hyperglycemic painful condition rather than true peripheral neuropathy. On the other hand, it is recommended that peripheral neuropathy in the original sense accompanied with destruction of peripheral nerve fibers be classified into two broad categories, symmetry disorder and asymmetry disorder (Non-Patent Literature 17). However, even in the case of hyperglycemic neuropathy, it is considered that the transmission pathway of pain from the periphery to the brain via the secondary pain sensation is reproduced. Therefore, the diabetic peripheral neuropathy according to the present invention is a concept that comprehensively includes hyperglycemic neuropathy.

The type of symmetry disorder is generically called symmetry polyneuropathy. Here, symmetric polyneuropathy is the most important target in the study field of diabetic peripheral neuropathy because unpleasant sensory symptoms and foot lesion formation not only interfere with quality of life (QOL), but also in severe cases, cardiovascular events due to autonomic dysfunction shorten the prognosis of life (Non-Patent Literature 17). Symmetric polyneuropathy forms the core of diabetic peripheral neuropathy according to the present invention.

Allodynia, hypersensitivity, burning pain, bean-like pain, numbness-like pain, etc. are often observed throughout neuropathic pain, and further pricking pain, spear-piercing pain, and twitching pain are often observed as characteristic pain in diabetic peripheral neuropathic pain.

### (4) Alleviation/treatment of diabetic neuropathic pain, etc.

### (4-1) Alleviation of neuropathic pain

In the present invention, the alleviation of neuropathic pain inclusively means that at least one of the intensity, frequency, pain duration, and property in neuropathic pain changes to a condition of reduction, decrease, shortening, amelioration, remission, or disappearance.

For example, when taking the medicament of the present invention or administering the active ingredient of the present invention reduces the intensity of neuropathic pain that occurred before taking or administering the medicament, it is understood that neuropathic pain is alleviated. The reduction in the intensity of neuropathic pain can be determined, for example, based on the difference in NRS scores before and after taking the medicament. The guidelines of the European Federation of Neurological Societies (EFNS) and the IASP's Neuropathic Pain Special Interest Group (NeuPSIG) prioritize reduction of pain intensity rather than multifaceted assessment of pain. The reduction or elimination of pain intensity can be preferably exemplified as the alleviation of neuropathic pain of the present invention.

Alternatively, for example, before taking the medicament of the present invention or before administering the active ingredient of the present invention, the nature of neuropathic pain is "allodynia", "hyperalgesia", or "pain pierced by a spear". Then, after taking the medicament of the present invention, when the nature of neuropathic pain just remains only "allodynia" and "hyperalgesia", it is understood that neuropathic pain is alleviated.

When taking the medicament of the present invention or administering the active ingredient of the present invention reduce the number of sites where neuropathic pain that occurred before such taking or administering, neuropathic pain can be understood to be alleviated.

### (4-2) Treatment of neuropathic pain

In the present invention, the treatment of neuropathic pain can mean the alleviation (or can be associated with alleviation) of neuropathic pain. For example, it may also mean that the alleviation of neuropathic pain can achieve sleep at night that is not disturbed by pain, elimination of pain at rest, elimination of pain during body movement, and the like.

Currently, NNT (a numerical value indicating how many patients need to be administered a drug in order to obtain one patient with 50% reduction in pain) is used as an index of therapeutic efficacy, and this index can also be used in the present invention. For example, it has been reported that tricyclic antidepressants are approximately 3.1 and calcium channel α2δ subunit binding agents are 4.7 for peripheral neuropathic pain (Patent Literature 6).

### (4-3) Alleviation of diabetic neuropathic pain

In the present invention, the above-mentioned idea of alleviating neuropathic pain can be applied to the alleviation of diabetic neuropathic pain.

The core of diabetic neuropathic pain is pain associated with symmetric polyneuropathy, but in pain associated with symmetric polyneuropathy, sensory neuropathy and autonomic neuropathy are generally clinically dominant, often followed by clinical symptoms of motor neuropathy with a delay. In general, positive symptoms (dust/stinging/tingling/burning pain) are observed in the early stage of the disease, and negative symptoms often occur as the disease progresses. When the diabetic neuropathic pain according to the present invention is pain associated with symmetric polyneuropathy, preferable examples include the said positive symptom as the pain.

### (5) Subject for administration

### (5-1) Patient with neuropathic pain of P2X7 receptor gain-of-function type, loss-of-function type, or unchanged function type

It has been reported that mutations in the human P2X7 receptor gene encoding the P2X7 receptor cause changes the function and expression of the P2X7 receptor, such as receptor transport, ATP binding, channel function and pore structure, resulting in leading to phenotypes such as loss-of-function type (LOF type) and gain-of-function type (GOF type). Further it is pointed out that mutations in the P2X7 receptor gene may be involved in pain sensitivity in humans (Non-Patent Literature 11). Specifically, many single nucleotide polymorphisms (SNPs) have been reported for the P2X7 receptor gene (Non-Patent Literatures 13 to 16).

Therefore, the patient according to the present invention is not particularly limited as long as the patient is a patient having a symptom of neuropathic pain (neuropathic pain patient), but includes a patient with the P2X7 receptor gain-of-function type, a patient with the P2X7 receptor unchanged function type, or a patient with the P2X7 receptor loss-of-function type. Preferable examples of the subject for administration include a patient with the P2X7 receptor gain-of-function type or a patient with the P2X7 receptor unchanged function type, particularly a patient with the P2X7 receptor gain-of-function type.

### (A) Single nucleotide polymorphism of P2X7 receptor gene

The single nucleotide polymorphism of the P2X7 receptor gene according to the present invention is not particularly limited, and examples include 11 types of single nucleotide polymorphisms described in Non-Patent Literature 15. Table 1 shows four typical single nucleotide polymorphisms that are associated with missense mutations and have been suggested to be associated with pain as a phenotype.

**[Table 1]**

| rsID | Nucleo tide mutati on | Mutation site for wild type in the nucleotide sequence shown in SEQ ID NO: 1 (*) | Amino acid mutation | Mutation site in the amino acid sequence shown in SEQ ID NO: 2 |
|---|---|---|---|---|
| rs2082 94 | C>T | 489 | His>Tyr | 155 |
| rs7958 311 | G>A | 835 | Arg>His | 270 |
| rs1718 119 | G>A | 1068 | Ala>Thr | 348 |
| rs3751 143 | A>C | 1513 | Glu>Ala | 496 |

| | | | | |
|---|---|---|---|---|
| (*) CDS: 27th to 1814th (The cording region is the number obtained by subtracting 26 from the corresponding number.) | | | | |

The function of the P2X7 receptor in which each single nucleotide polymorphism occurred and the frequency of the polymorphism (minor allele frequency) have been reported as follows (Non-Patent Literatures 13 and 15).

**[Table 2]**

| rsID | Nucleotide mutation | Function (Non-Patent Literatures 13 and 15) | Minor allele frequency (Non-Patent Literature 15) |
|---|---|---|---|
| rs208294 | C>T | gain-of-function type (GOF type) | T=0.439 |
| rs7958311 | G>A | loss-of-function type (LOF type) | A=0.255 |
| rs1718119 | G>A | gain-of-function type (GOF type) | A=0.400 |
| rs3751143 | A>C | loss-of-function type (LOF type) | C=0.175 |

### (B) Patient with the P2X7 receptor gain-of-function type (GOF type)

In the present invention, the P2X7 receptor gain-of-function type diplotype is characterized by the P2X7 receptor gene in which both of the two P2X7 receptor genes (alleles) located on each of the homologous chromosomes derived from a certain patient have gain-of-function type mutations (the P2X7 receptor gain-of-function type haplotype), and as a result, it means a condition in which the function of the mutated P2X7 receptor is enhanced.

The patient with the P2X7 receptor gain-of-function type (GOF type) is a patient showing a diplotype (haplotype pair) in which a gain-of-function type mutation occurs in the locus of the P2X7 receptor gene, and as a result, when the mutated P2X7 receptor is in a condition of enhanced function, the patient can be regarded as a patient with the P2X7 receptor gain-of-function type (GOF type patient). More specifically, when both of the two P2X7 receptor genes (alleles) located on each of the patient-derived homologous chromosomes show the P2X7 receptor GOF type haplotype shown below, the patient can be called a GOF-type patient.

Examples of GOF-type haplotypes include haplotypes that show a degree of variation against the function of wild-type haplotypes of 160% or more, more preferably 1700 or more, further more preferably 180% or more, and particularly preferably 190% or more, and most preferably 200% or more as the function of the P2X7 receptor measured by the *in vitro* assay (Yo-Pro-1 assay method). Examples of the GOF type haplotype include the haplotypes (Nos. 1 to 4) shown in Table 3 in which the function of the P2X7 receptor is 160% or more.

For example, when the P2X7 receptor gene located on one of the homologous chromosomes is the No. 1 haplotype in Table 3, and the P2X7 receptor gene located on the other chromosome is the No. 3 haplotype in Table 3 in a certain patient, it can be determined that the patient is a patient with the P2X7 receptor GOF type.

### (C) The patient with the P2X7 receptor loss-of-function type (LOF type)

In the present invention, the P2X7 receptor loss-of-function type diplotype means a condition in which the function of the P2X7 receptor encoded by the P2X7 receptor gene causing the P2X7 receptor loss-of-function type mutation is attenuated, wherein the P2X7 receptor loss-of-function type diplotype comprises, in two P2X7 receptor genes (alleles) located on each homologous chromosome from a certain patient,
1) the P2X7 receptor gene in which both of them have P2X7 receptor loss-of-function type mutations (P2X7 receptor LOF type haplotype), or
2) one is the P2X7 receptor gene that causes one P2X7 receptor loss-of-function type mutation (P2X7 receptor LOF type haplotype), and the other is the P2X7 receptor unchanged function type gene (P2X7 receptor standard type haplotype).

When the patient with the P2X7 receptor loss-of-function type (LOF type) is either 1) a patient shows a diplotype (haplotype pair) that has a LOF type mutation at the locus of the P2X7 receptor gene, or
2) a patient in which one chromosome shows a haplotype with a loss-of-function type mutation at the locus of the P2X7 receptor gene and the other chromosome shows a P2X7 receptor standard type haplotype, and has a condition in which the function of the mutated P2X7 receptor is attenuated, such patient can be considered as a patient with the P2X7 receptor loss-of-function type (LOF type patient). More specifically, when 1) both of the two P2X7 receptor genes (alleles) located on each of the patient-derived homologous chromosomes exhibit the P2X7 receptor LOF type haplotype shown below, or 2) one shows the LOF type haplotype and the other shows the standard type haplotype, such patient can be regarded as a LOF patient.

Examples of LOF-type haplotypes include haplotypes that show a degree of variation against the function of wild-type haplotypes of 80% or less, more preferably 70% or less, further more preferably 60% or less, and particularly preferably 50% or less, and most preferably 40% or less as the function of the P2X7 receptor measured by the *in vitro* assay (Yo-Pro-1 assay method). Examples of the LOF type haplotype include the haplotypes (Nos. 13 to 16) shown in Table 3 in which the function of the P2X7 receptor shows 40% or less.

Examples of standard type haplotypes include haplotypes that show the upper limit of 200% or less, more preferably 190% or less, further more preferably 180% or less, and particularly preferably 170% or less, and most preferably 160% or less, 150% or less, or 1400 or less and the lower limit of 40% or more, more preferably 50% or more, further more preferably 60% or more, and particularly preferably 70% or more, and most preferably 80% or more, 90% or more, or 100% or more, as the function of the P2X7 receptor measured by the *in vitro* assay (Yo-Pro-1 assay method). Examples of the standard type haplotype include the haplotypes (Nos. 7 to 12) shown in Table 3 in which the function of the P2X7 receptor is more than 40% and less than 120% or less.

For example, when the P2X7 receptor gene located on one of the homologous chromosomes is the No. 15 haplotype in Table 3, and the P2X7 receptor gene located on the other chromosome is the No. 16 haplotype in Table 3 in a certain patient, such patient is determined to be a patient with the P2X7 receptor loss-of-function type.

### (D) Patient with the P2X7 receptor unchanged function type

Further, in the present invention, the patient with the P2X7 receptor unchanged function type (standard type) is not particularly limited as long as the patient is a patient other than the patient with P2X7 receptor gain-of-function type (GOF type) or the patient with the P2X7 receptor loss-of-function type (LOF type). Typical examples of the patient with the P2X7 receptor unchanged function type include a patient in which both of the two P2X7 receptor genes (alleles) located on each homologous chromosome from a certain patient are wild-type P2X7 receptor genes.

### (E) Assessment method for the effect of a combination of single nucleotide mutations in the human P2X7 receptor gene on the function of the P2X7 receptor

When a mutation occurs in the P2X7 receptor gene, the function of the mutated P2X7 receptor can be easily confirmed by a person skilled in the art by a conventional method.

For example, the function of the P2X7 receptor can be measured by measuring the variation of the intracellular Ca²⁺ concentration when stimulated with BzATP using a fluorescent indicator having high Ca²⁺ sensitivity in cells stably expressing the (Patent Literature 5). Alternatively, the assay system in which the nucleic acid fluorescence staining reagent YO-PRO-1 is incorporated into cells stably expressing the P2X7 receptor can be used together with or instead.

The present inventors assessed the effect of the combination of the single nucleotide polymorphism in the P2X7 receptor gene on the function of P2X7 receptor for all combinations of the presence or absence of mutation (2 X 2 X 2 X 2 = 16 patterns) in the four types of single nucleotide polymorphisms mentioned above (Example 4). Specifically, the P2X7 receptor expressed by the combination of rs208294 = C, rs7958311 = G, rs17181119 = G, and rs3751143 = A was set as a wild-type haplotype, and the value of the said wild-type haplotype P2X7 receptor stimulated with BzATP 300 µM was measured on each measuring plate as a reference. With that value as 100%, the function of each combination of P2X7 receptors was assessed. A reference well was also placed on the measuring plate for the wild-type haplotype and the function of the wild-type haplotype was shown with the measured value of the reference well as 100%. The average value measured three times with n = 3 was shown as a result.

**[Table 3]**

| NO. | rsID and its nucleotide (*) | | | | Function of P2X7 receptor in the *in vitro* assay (Yo-Pro-1 assay method) (%) |
|---|---|---|---|---|---|
| | rs208294 | rs7958311 | rs1718119 | rs3751143 | |
| 1 | T(good) | A(poor) | A(good) | A | 294.8 |
| 2 | T(good) | G | A(good) | A | 287.4 |
| 3 | C | G | A(good) | A | 249. 6 |
| 4 | C | A(poor) | A(good) | A | 224.0 |
| 5 | T(good) | A(poor) | G | A | 154.7 |
| 6 | T(good) | G | G | A | 146.0 |
| 7 | T(good) | A(poor) | A(good) | C(poor) | 118.8 |
| 8 | C | G | G | A | 110.3 |
| 9 | T(good) | G | A(good) | C(poor) | 98.7 |
| 10 | C | A(poor) | A(good) | C(poor) | 97.7 |
| 11 | C | A(poor) | G | A | 93.7 |
| 12 | C | G | A(good) | C(poor) | 82.9 |
| 13 | T(good) | G | G | C(poor) | 35.8 |
| 14 | T(good) | A(poor) | G | C(poor) | 32.4 |
| 15 | C | G | G | C(poor) | 21.4 |
| 16 | C | A(poor) | G | C(poor) | 21.0 |

| | | | | | |
|---|---|---|---|---|---|
| (*) According to Non-Patent Literatures 13 and 15, "good" or "poor" is added to the gain-of-function type mutation or the loss-of-function type mutation, respectively. | | | | | |

Based on these results, taking general common thought by a person skilled in the art such as experimental errors and assay characteristics into consideration, it is defined in Example 4 that Nos. 1 to 4 showing the P2X7 receptor function of 200% or more is a P2X7 receptor gain-of-function type haplotype (GOF type haplotype); Nos. 7 to 12 showing functions within a range of +/- 20% centered in the functions of the wild type haplotype is a standard haplotype; and Nos. 13 to 16 showing the P2X7 receptor function of less than 40% is a loss-of-function type haplotype (LOF type haplotype).

### (5-2) Patient with diabetic peripheral neuropathy, etc.

The subject for administrating the medicament according to the present invention is not particularly limited as long as the person is a patient with diabetic peripheral neuropathy or the like, and examples may include a person suffering from any of "hyperglycemic neuropathy", "symmetric polyneuropathy", "local and multifocal neuropathy", and "mixed type" according to the Thomas classification. In addition, the background of the patient such as age, gender, height, weight, comorbidities, and underlying diseases is not particularly limited, either.

Further, the subject for administrating the medicament according to the present invention may be a patient with diabetic peripheral neuropathy or the like showing the P2X7 receptor gain-of-function type, the P2X7 receptor unchanged function type, or the P2X7 receptor loss-of-function type. Preferable examples of the subject for administration include a patient with diabetic peripheral neuropathy, etc., showing the P2X7 receptor gain-of-function type or the P2X7 receptor unchanged function type, particularly the P2X7 receptor gain-of-function type.

### (6) Administration method (administration route/ administration frequency/dose, etc.)

The route of administration of the medicament of the present invention is not particularly limited, and may be oral administration or parenteral administration. Examples of parenteral administration include rectal administration, transdermal administration, subcutaneous administration, intradermal administration, intramuscular administration, eye drop administration, nasal drop administration, intravenous administration, intrathecal administration and the like.

The dose and frequency of administration of the medicament of the present invention can be determined by a doctor, etc. For example, the dose and frequency of administration can be appropriately adjusted depending on the active ingredient, the progress of the disease condition and disorder, the age, the weight, the underlying disease, the cause of disease, etc.

When the medicament of the present invention is used [for example, when a compound described in Patent Literature 5 such as Compound 7 or a salt thereof is used as a compound that attenuates the function of the P2X7 receptor (active ingredient)], preferred examples of dosage/usage include the active ingredient in the dose range of about 0.05 mg to about 3000 mg, about 1 mg to about 1000 mg, or about 10 mg to about 500 mg, once daily or at least once daily, *e.g*., twice , 3 or 4 times daily.

When the medicament of the present invention is administered to a patient, it may be confirmed whether or not the patient exhibits the P2X7 receptor gain-of-function type or the P2X7 receptor loss-of-function type by, for example, genotyping.

In general, genotyping is known as a method for detecting genotype differences (allele combinations, etc.) by identifying the DNA sequence of a certain individual by DNA sequencing, etc. and then comparing the DNA sequence with that of another individual, and can be carried out by a conventional method (Non-Patent Literature 15).

Specifically, for example, a sample derived from the patient (e.g., peripheral venous blood) is collected, and a sample for genotyping can be prepared by extracting and amplifying DNA from the collected sample by a conventional method. Then, the prepared sample is donated on a chip on which a known DNA fragment is immobilized can be genotyped by hybridization of the DNA in the sample with the known DNA fragment.

Alternatively, for example, collecting a sample derived from the patient (e.g., peripheral venous blood), extracting DNA from the collected sample by a conventional method, then mixing a sample containing the extracted DNA, a set of PCR primers, and a TaqMan (registered trademark) probe corresponding to a single nucleotide polymorphism of DNA (including a complementary sequence of the mutated DNA), and finally performing PCR can detect the single nucleotide polymorphism of the probe.

Examples of the single nucleotide mutation of the DNA include rS208294 (H155Y), rS7958311 (R270H), rS17181119 (A348T), and rS3751143 (E496A). Alternatively, preferable examples of the single base mutation of the DNA (0 to 4 single base mutations in the P2X7 gene) include the single base mutation described in Nos. 1 to 16 in Table 3.

### (7) Administration method (combination use)

According to the treatment guidelines presented by the Japan Neurotherapy Society in view of approval and availability in Japan with reference to the guidelines presented by the International Pain Society in 2007, examples of the compound include tricyclic antidepressants (TCA), serotonin-noradrenaline reuptake inhibitors (SNRIs), calcium channel α2δ subunit binding drugs, and local anesthetics (topical lidocaine) as first-line drugs; opioid analgesics as second-line drugs; and selective serotonin reuptake inhibitors (SSRIs), antiarrhythmics, and capsaicin as third-line drugs (Patent Literature 6). The IASP recommendation can be also referred (Lancet Neurol. 2015 Feb; 14 (2): 162-73).

In the present invention, the active ingredient of the present invention can be used in combination with a different pain-therapeutic compound (*e.g.,* a compound for alleviating neuropathic pain different from the compound that attenuates the function of the P2X7 receptor, which is exemplified in the Embodiment 2 described later). Preferable examples of compounds that can be used in combination are not particularly limited, and include the following compounds.

### (7-1) Tricyclic antidepressant (TCA)

Amitriptyline, imipramine, clomipramine, nortriptyline, and desipramine (Non-Patent Literature 1)

### (7-2) Serotonin-noradrenaline reuptake inhibitor (SNRI) Duloxetine and venlafaxine (Non-Patent Literature 1)

### (7-3) Calcium channel α2δ subunit binding drug Pregabalin and gabapentin (Non-Patent Literature 1)

### (7-4) Local anesthetics Topical lidocaine (Patent Literature 6)

### (7-5) Vaccinia virus inoculated rabbit inflamed skin extract (Non-Patent Literature 1)

### (7-6) Opioid analgesics

Tramadol, tramadol/acetaminophen combination drug, buprenorphine, fentanyl, and morphine

A medicament containing at least one of these compounds may be administered together with or after a period of time, and a combination drug containing at least one of these compounds and the active ingredient of the present invention may be taken. The usage of these compounds can follow a conventional method (Non-Patent Literature 1).

### (8) Medicament

The medicament may be manufactured by mixing an effective amount of the active ingredient of the present invention with various pharmaceutical additives suitable for the formulation, such as excipients, binders, disintegrants, lubricants and the like. Furthermore, the said medicament can be for pediatric patients, geriatric patients, or serious cases by appropriately changing the effective amount of the active ingredient of the present invention, formulation and/or various pharmaceutical additives.

### 2. Embodiment 2

Another embodiment of the medicament of the present invention is a medicament for alleviating neuropathic pain, which is administered to a patient in which the function of the P2X7 receptor is enhanced.

One embodiment of the therapeutic method of the present invention is a method for alleviating neuropathic pain, which comprises administering a compound for alleviating neuropathic pain to a patient in which the function of the P2X7 receptor is enhanced.

One embodiment of the therapeutic method of the present invention is a method for treating neuropathic pain, which comprises administering a compound for alleviating neuropathic pain to a patient in which the function of the P2X7 receptor is enhanced.

### (1) Active ingredient (compound for alleviating neuropathic pain)

The active ingredient in the present invention may be a compound of the present invention that "attenuates the function of the P2X7 receptor" (*e.g.,* the compound exemplified in the above Embodiment 1), and a compound for alleviating neuropathic pain different from the same compound (Non-Patent Literature 1, Non-Patent Literature 3, and Patent Literature 6). A compound that "attenuates the function of the P2X7 receptor" and a compound for alleviating neuropathic pain different from the same compound may be referred to as a compound for alleviating neuropathic pain.

As described above, examples of the compound for alleviating neuropathic pain different from the said same compound include tricyclic antidepressants (TCA), serotonin-noradrenaline reuptake inhibitors (SNRIs), calcium channel α2δ subunit binding agents, local anesthetics (topical lidocaine), opioid analgesics, selective serotonin reuptake inhibitors (SSRIs), antiarrhythmics, capsaicin (Non-Patent Literature 6). Specific examples include the following compounds.

**[Table 4]**

| NO. | Class | Compound |
|---|---|---|
| 1 | tricyclic antidepressant (TCA) | amitriptyline |
| 2 | | imipramine |
| 3 | | clomipramine |
| 4 | | nortriptyline |
| 5 | | desipramine |
| 6 | | doxepin |
| 7 | serotonin-noradrenaline reuptake inhibitor (SNRI) | duloxetine |
| 8 | | venlafaxine |
| 9 | | imipramine |
| 10 | | milnacipran |
| 11 | selective serotonin reuptake inhibitor (SSRI) | citalopram |
| 12 | | fluoxetine |
| 13 | | sertraline |
| 14 | | escitalopram |
| 15 | | paroxetine |
| 16 | | fluvoxamine maleate |
| 17 | calcium channel α2δ subunit binding drug | pregabalin |
| 18 | | gabapentin |
| 19 | local anesthetic | lidocaine |
| 20 | | cocaine |
| 21 | | capsaicin |
| 22 | | benzocaine |
| 23 | | tetracaine |
| 24 | | butamben |
| 25 | Vaccinia virus inoculated rabbit inflamed skin extract | |
| 26 | opioid analgesic | tramadol |
| 27 | | buprenorphine |
| 28 | | fentanyl |
| 29 | | remifentanil |
| 30 | | morphine (hydromorphone, etc.) |
| 31 | | oxycodone |
| 32 | | hydrocodone |
| 33 | | meperidine (pethidine) |
| 34 | | methadone |
| 35 | | codeine |
| 36 | | nalbuphine |
| | | hydrochloride |
| 37 | | oxymorphone |
| 38 | | tapentadol |
| 39 | | butorphanol |
| 40 | sodium channel inhibitor | lamotrigine |
| 41 | | oxycarbazepine |
| 42 | | carbamazepine |
| 43 | NMDA-type glutamate receptor inhibitor | ketamine |
| 44 | others | antipyrine |
| 45 | | l-menthol |
| 46 | | Botulinum toxin type A |

These compounds can be used alone, and for example, they can be used in combination as appropriate in the form of a combination drug.

### (2) Alleviation of neuropathic pain

The "neuropathic pain" in Embodiment 1 is applied to the "neuropathic pain" in the present invention. The "alleviation of neuropathic pain" in Embodiment 1 is applied to the "alleviation of neuropathic pain" in the present invention.

As described above, neuropathic pain is classified into two broad categories, peripheral neuropathic pain and central neuropathic pain, depending on the site of nerve injury. Examples include peripheral neuropathic pain as neuropathic pain in the present invention, and examples of the alleviation of peripheral neuropathic pain include as the alleviation of neuropathic pain in the present invention.

Examples includes postherpetic neuralgia pain (PHN), post-traumatic peripheral neuropathic pain, diabetic peripheral neuropathic pain (DPNP), trigeminal neuralgia (TGN), neuropathic pain after spinal code industry, chemotherapy-induced peripheral neuropathic pain (CIPNP; or also called CIPN), and post-surgical pain (PSP) as neuropathic pain according to the present invention.

### (3) Treatment of neuropathic pain

The "treatment of neuropathic pain" in Embodiment 1 is applied to the "treatment of neuropathic pain" in the present invention.

As mentioned above, neuropathic pain is classified into two broad categories, peripheral neuropathic pain and central neuropathic pain, depending on the site of nerve injury. Examples of the treatment of peripheral neuropathic pain include as the treatment of neuropathic pain in the present invention.

### (4) Subject for administration (patient with enhanced P2X7 receptor function)

One of the features of the present invention is that the patient is a neuropathic pain patient whose function of the P2X7 receptor is enhanced. Here, the "neuropathic pain patient with the P2X7 receptor gain-of-function type (GOF type patient)" in Embodiment 1 can be applied to the "patient with enhanced P2X7 receptor function".

Specific examples of the patient of the present invention include a patient showing a diplotype (haplotype pair) in which a gain-of-function type mutation occurs in the locus of the P2X7 receptor gene, and the resulting patient is in a condition of enhanced function of the P2X7 receptor in which the mutation occurs. Preferable examples of the gain-of-function type mutation include a gain-of-function type mutation in the P2X7 receptor (GOF type haplotype) according to No. 1 to 4 in Table 3.

Whether or not the patient to be administered is a patient with enhanced P2X7 receptor function can be determined by, for example, genotyping using a sample derived from the patient to identify the genotype (two alleles) of the P2X7 receptor gene, followed by measuring the function of the P2X7 receptor by measuring the change in intracellular Ca²⁺ concentration when stimulated with BzATP using a Ca²⁺ sensitive fluorescent indicator in cells stably expressing the P2X7 receptor gene. Alternatively, the assay system in which the nucleic acid fluorescence staining reagent YO-PRO-1 is incorporated into cells stably expressing the P2X7 receptor can be used together with or instead.

### (5) Administration method (administration route/administration frequency/dose/combination, etc.)

As the administration method in the present invention, the "administration method (including administration route/administration frequency/dose, etc.)" and "administration method (including combination use)" in Embodiment 1 can be applied. The "compound that attenuates the function of the P2X7 receptor" (active ingredient) in Embodiment 1 may be applied in place of the compound of the present invention (i.e., a compound for alleviating neuropathic pain).

### (6) Medicament

The "medicament" in Embodiment 1 can be applied to the medicament in the present invention.

### [Example]

The present invention will be explained more specifically by means of examples. However, the present invention is not intended to be bound to the following examples, and the present invention can be carried out in any embodiments within the scope that does not depart from the spirit of the present invention.

### [Example 1]

### (Study for assessing the tissue distribution of the P2X7 receptor antagonist compound)

### 1. Study for pharmacokinetics in blood

### (1) Study method

Rats (strain: Crl: CD (SD), male, 7 weeks old, with diet) were orally administered a methylcellulose suspension containing a P2X7 receptor antagonist compound (Compound 7 or Compound 10). Then blood was collected from rats 15 and 30 minutes, 1, 2, 4, 8 and 24 hours after the administration, and the concentration of the compound contained in the collected blood was measured. The dose of the compound per dose was 30 mg/kg, and the concentration of the compound in the suspension was 3 mg/mL. Administration was carried out once daily for 4 consecutive days.

### (2) Study result

The variations of a blood level of the compound with the passage of time were analyzed by a conventional method, and the pharmacokinetic parameters were estimated. The result is shown in the table below.

**[Table 5]**

| | Compound 7 | | Compound 10 | |
|---|---|---|---|---|
| Parameter | Day 1 of administra tion | Day 4 of administra tion | Day 1 of administra tion | Day 4 of administra tion |
| Tₘₐₓ(hr) | 0.333 | 0.583 | 0.333 | 0.250 |
| Cₘₐₓ(µmol/L) | 18.5 | 14.7 | 9.54 | 8.27 |
| AUCₗₐₛₜ(hr*µm ol/L) | 80.1 | 77.4 | 48.8 | 45.6 |

### 2. Study for assessing the central tissue distribution

### (1) Study method

Rats (strain: Crl: CD (SD), male, 7 weeks old, with diet) were orally administered a methylcellulose suspension containing a P2X7 receptor antagonist compound (Compound 7 or Compound 10). Then central tissue was collected from rats 1 hour after its administration, and the compound distribution to the collected central tissue was assessed. The dose of the compound per dose was 30 mg/kg, and the concentration of the compound in the suspension was 3 mg/mL.

### (2) Study result

The study was carried out with n = 2 or n = 3. The study result is shown in the table below.

**[Table 6]**

| | Compound 7 | Compound 10 |
|---|---|---|
| Tissue | Mean value of n = 2 | Mean value of n = 3 |
| Plasma concentration (umol/L) | 19.3 | 5.08 |
| Brain concentration (nmol/g tissue) | 23.6 | 9.22 |
| Spinal cord concentration (nmol/g tissue) | 27.7 | 11.4 |

This orally administered compound showed the sufficient central tissue distribution.

Based on the above result, it is considered that even if the compound is another P2X7 receptor antagonist compound, as long as the compound is a low molecular weight compound, the compound a high possibility to exhibit the sufficient central tissue distribution.

### [Example 2]

### (Study for confirming the effect of the compound on pain in rat chronic stenosis injury model (CCI model or Bennett model)

In order to confirm the effect of Compound 7 on neuropathic pain, the effect of Compound 7 on hyperalgesia caused by mechanical stimulation occurring in CCI model rats was investigated. Compound 7 was orally administered to rats as an investigational drug, and the following studies were carried out.

### (1) Preparation of CCI model rat

As an experimental animal, a 7-week-old Sprague Dawley male rat (220.4 to 328.0 g) was used. The surgery was performed according to the procedure described in Bennett et al., Pain, 1988, 33, 87-107. Under isoflurane anesthesia, the right sciatic nerve was exposed *via* a blunt incision in the biceps femoris. Proximal to the bifurcation of the sciatic nerve, the nerve was freed of adhering tissue and 4 loose ligatures of 4-0 silk were tied around the nerve. Spacing between ligatures was approximately 1mm. The muscle was sutured in layers, and the skin closed with silk suture.

### (2) Administration of investigational drug

The experimental animals were composed of four groups: a solvent administration group and each administration group of either 2, 10 or 50 mg/kg of Compound 7 (Compound 7-administration group). Compound 7 was orally administered to the Compound 7-administration group in a single dose 3 hours before the measurement test performed 12 days after CCI surgery. Only the solvent of Compound 7-administration group was similarly administered to the solvent administration group.

### (3) Statistical processing

Statistical processing of the result was performed using a paired t-test for comparison between the two groups, and a significance level of 5% or less on both sides was considered significant. For comparison among the four groups, a parametric Williams' test was used for the Compound 7-administered group with the solvent group as a control, and a significance level of 2.5% or less on one side was considered to be significantly different.

### (4) Randall-Selitto test

The above rats were subjected to a measurement based on the paw pressure test described in Randall LO et al., Arch. Int. Pharmacodyn. Ther., 1957, 111, 409-419 (Randall-Selitto test). Namely, the right hind paw was gradually increasingly pressurized with a pressure stimulation analgesic effect analyzer, and the pressure at which each rat showed an abnormal phonation reaction or escape reaction was determined as the pain threshold value.

The baseline threshold value was measured before CCI surgery, and then the pain threshold value was measured 12 days after surgery. After confirming that the pain threshold value was sufficiently lowered, the measurement test was carried out.

On the 12th day after the surgery, the pain threshold value was significantly lower than that before the CCI surgery. In addition, in the Compound 7-administration group, the decrease of the threshold value observed in CCI surgery was significantly increased in all the administration groups.

From the above result, it was confirmed that Compound 7 was an example having the therapeutic effect on mechanical hyperalgesia occurring in CCI model rats.

### [Example 3]

### (Study for confirming the effect of the compound on pain in rat spinal nerve ligation model (SNL or Chung model)

In order to confirm the effect of Compound 7 on neuropathic pain, the effect of Compound 7 on allodynia caused by mechanical stimulation occurring in SNL model rats was investigated. Compound 7 was orally administered to rats as a test drug, and the following tests were carried out.

### (1) Preparation of SNL model rat

As an experimental animal, a 6-week-old Sprague Dawley male rat (180 to 210 g) was used. The surgery was performed according to the procedure described in Chung et al., Pain, 1992, 50, 355-363. Under isoflurane anesthesia, a longitudinal incision was made in the dorsal skin from the thoracic spine to the sacral spine level. After incision and isolation of the paravertebral muscles, the L5 and L6 transverse processes were removed to expose the L5 and L6 spinal nerves. These nerves were ligated with 5-0 silk suture. Sham operated animals were treated identically with the exception that the nerves were not ligated. The muscle was sutured in layers, and the skin was closed with silk suture.

### (2) Administration of investigational drug

The experimental animals consisted of 6 groups: a sham operation group, a solvent administration group, and each administration group of either 0.4, 2, 10, or 50 mg/kg of Compound 7 (Compound 7-administration group). Compound 7 was orally administered to the Compound 7-administration group once a day for 7 days from the 15th day after SNL surgery. Only the solvent of Compound 7-administration group was similarly administered to the sham surgery group and the solvent administration group.

### (3) Statistical processing

Statistical processing of the result was performed using Student's t-test for comparison between the two groups, and then for comparison among the five groups, a Dunnett's test was used for the Compound 7-administered group with the solvent group as the control. A significance level of 5% or less was considered to be significantly different.

### (4) Dynamic Plantar Aesthesiometer test

The pain threshold for mechanical stimulation in the above rats, was measured using a Dynamic Plantar Aesthesiometer (manufactured by Ugo Basile). Namely, using a Dynamic Plantar Aesthesiometer, the left hind paw was gradually increasingly pressurized at 30 g/40 seconds, and the pressure at which each rat showed an escape reaction was determined as the pain threshold value.

In the solvent administration group, the pain threshold value was significantly decreased as compared with the sham surgery group, whereas in the 50 mg/kg administration group of Compound 7, the decrease of the threshold value observed in SNL surgery was significantly increased.

From the above result, it was confirmed that Compound 7 was an example having the therapeutic effect on the pain occurring in SNL model rats.

### [Example 4]

### (Study for assessing the effect of the combination of single nucleotide mutations in the human P2X7 receptor gene on the P2X7 receptor function)

### 1. Study method

Human embryonic kidney cells 293 (HEK293) was cultured on a 10 cm dish (plate) by a conventional method so as to be 3e + 6 cells/dish. Each plasmid incorporating each of the 16 types of human P2X7 receptor gene mutants (Nos. 1 to 16 in the table below) prepared in advance by a conventional method was transfected into HEK293 24 hours after the start of culture, and then culturing was continued on a 96-well plate for an additional 24 hours in a conventional manner to 5e + 4 cells/well. After the cell culture medium was washed with a buffer solution, an appropriate amount of a 2 µM concentration of a coloring dye (Yo-Pro-1) solution or a calcium 5 and probenecid (anion transporter inhibitor) solution was timely added to the culture. The resulting culture was incubated for 10 or 60 minutes and subjected to the assay.

**[Table 7]**

| NO. | rsID and its nucleotide (*) | | | |
|---|---|---|---|---|
| | rs208294 | rs7958311 | rs1718119 | rs3751143 |
| 1 | T(good) | A (poor) | A(good) | A |
| 2 | T(good) | G | A(good) | A |
| 3 | C | G | A(good) | A |
| 4 | C | A (poor) | A(good) | A |
| 5 | T(good) | A (poor) | G | A |
| 6 | T(good) | G | G | A |
| 7 | T(good) | A (poor) | A(good) | C(poor) |
| 8 | C | G | G | A |
| 9 | T(good) | G | A(good) | C(poor) |
| 10 | C | A (poor) | A(good) | C(poor) |
| 11 | C | A (poor) | G | A |
| 12 | C | G | A(good) | C |
| 13 | T(good) | G | G | C (poor) |
| 14 | T(good) | A (poor) | G | C (poor) |
| 15 | C | G | G | C(poor) |
| 16 | C | A (poor) | G | C(poor) |

| | | | | |
|---|---|---|---|---|
| (*) According to Non-Patent Literatures 13 and 15, "good" or "poor" is added to the gain-of-function type mutation or the loss-of-function type mutation, respectively. | | | | |

The Yo-Pro-1/calcium 5 uptake assay was performed using a FLIPRTETRA (registered trademark) cell-based screening system (manufactured by Molecular Devices).

The assay conditions are as follows:
■ Experimental Setup Parameter
   - Excitation Wavelength (nm): 470-495
   - Emission Wavelength (nm): 515-575
   - Camera Gain: 100
   - Exposure Time (s): 0.4
   - Excitation Integrity (%): 80

For the Yo-Pro-1 uptake assay, an HBSS buffer containing 20 mM HEPES was used as an assay buffer measured for 40 minutes after the addition of the BzATP-PBS solution.

The calcium 5 uptake assay was measured for 6.7 minutes after the addition of BzATP-PBS solution. As the buffer for the assay, an HBSS buffer containing 1.8 mM CaCl₂, 1 mM MgCl₂, and 20 mM HEPES was used.

The P2X7 receptor expressed in the combination of rs208294 = C, rs7958311 = G, rs17181119 = G, and rs3751143 = A was designated as a wild-type haplotype. The value of the said wild-type haplotype P2X7 receptor stimulated with BzATP 300 µM was measured on each measuring plate as a reference. With that value as 100%, the function of each combination of P2X7 receptors was assessed. A reference well was also placed on the measuring plate for the wild-type haplotype, and the function of the wild-type haplotype was shown with the measured value as 100%. The average value measured three times with n = 3 was shown as a result.

### 2. Study result

**[Table 8]**

| NO. | rsID and its nucleotide (*) | | | | Function of P2X7 receptor in the *in vitro* assay (Yo-Pro-1 assay method) (%) |
|---|---|---|---|---|---|
| | rs208294 | rs7958311 | rs1718119 | rs3751143 | |
| 1 | T(good) | A(poor) | A(good) | A | 294.8 |
| 2 | T(good) | G | A(good) | A | 287.4 |
| 3 | C | G | A(good) | A | 249.6 |
| 4 | C | A(poor) | A(good) | A | 224.0 |
| 5 | T(good) | A(poor) | G | A | 154.7 |
| 6 | T(good) | G | G | A | 146.0 |
| 7 | T(good) | A(poor) | A(good) | C(poor) | 118.8 |
| 8 | C | G | G | A | 110.3 |
| 9 | T(good) | G | A(good) | C(poor) | 98.7 |
| 10 | C | A(poor) | A(good) | C(poor) | 97.7 |
| 11 | C | A(poor) | G | A | 93.7 |
| 12 | C | G | A(good) | C(poor) | 82.9 |
| 13 | T(good) | G | G | C(poor) | 35.8 |
| 14 | T(good) | A(poor) | G | C(poor) | 32.4 |
| 15 | C | G | G | C(poor) | 21.4 |
| 16 | C | A(poor) | G | C(poor) | 21.0 |

| | | | | | |
|---|---|---|---|---|---|
| (*) According to Non-Patent Literatures 13 and 15, "good" or "poor" is added to the gain-of-function type mutation or the loss-of-function type mutation, respectively. | | | | | |

**[Table 9]**

| NO. | rsID and its nucleotide (*) | | | | Function of P2X7 receptor in the *in vitro* assay (Calcium 5 assay method) (%) |
|---|---|---|---|---|---|
| | rs208294 | rs7958311 | rs1718119 | rs3751143 | |
| 1 | T(good) | A (poor) | A(good) | A | 142.7 |
| 2 | T(good) | G | A(good) | A | 128.3 |
| 3 | C | G | A(good) | A | 130.0 |
| 4 | C | A (poor) | A(good) | A | 130.2 |
| 5 | T(good) | A (poor) | G | A | 120.8 |
| 6 | T(good) | G | G | A | 113.3 |
| 7 | T(good) | A(poor) | A(good) | C(poor) | 91.3 |
| 8 | C | G | G | A | 102.5 |
| 9 | T(good) | G | A(good) | C(poor) | 76.2 |
| 10 | C | A (poor) | A(good) | C(poor) | 86.2 |
| 11 | C | A (poor) | G | A | 107.5 |
| 12 | C | G | A(good) | C(poor) | 72.5 |
| 13 | T(good) | G | G | C(poor) | 50.8 |
| 14 | T(good) | A (poor) | G | C(poor) | 59.6 |
| 15 | C | G | G | C(poor) | 43.9 |
| 16 | C | A (poor) | G | C(poor) | 49.9 |

| | | | | | |
|---|---|---|---|---|---|
| (*) According to Non-Patent Literatures 13 and 15, "good" or "poor" is added to the gain-of-function type mutation or the loss-of-function type mutation, respectively. | | | | | |

From the result of the *in vitro* assay (Yo-Pro-1 assay method) (Table 8), it is defined that Nos. 1 to 4 showing the P2X7 receptor function of 200% or more is a P2X7 receptor gain-of-function type haplotype (GOF type haplotype); Nos. 7 to 12 showing functions within a range of +/- 20% centered in the functions of the wild type haplotype is a standard haplotype; and Nos. 13 to 16 showing the P2X7 receptor function of less than 40% is a loss-of-function type haplotype (LOF type haplotype). In addition, it is thought that this result shows that when rs208294 (C> T; GOF type mutation) is compared with rs1718119 (G> A; GOF type mutation), rs1718119 (G> A) is a mutation that induces GOF more strongly. Furthermore, it is thought that this result shows that when comparing rs7958311 (G> A; LOF type) with rs3751143 (A> C; LOF type), rs3751143 (A> C) is a mutation that induces loss-of-function more strongly.

It is shown that the result of the P2X7 receptor function in the *in vitro* assay (Calcium 5 assay method) (Table 9) are consistent with or support the classification of the GOF type haplotype/standard halo type/LOF type haplotype according to the order of the size of the GOF haplotype and LOF haplotype receptor functions in the Yo-Pro-1 assay.

### [Example 5]

### (Study for assessing the effect of single nucleotide mutations in the human P2X7 receptor gene on neuropathic pain)

### 1. Study method

### (1) Case extraction method

Using a database (Biobank owned by Vanderbilt University Medical Center; available from Nashville Biosciences), which contains various and enormous clinical information and genetic information on patients with neuropathic pain, study for assessing the effect of single nucleotide mutations in the human P2X7 receptor gene on neuropathic pain was carried out. This study is sometimes referred to as genotyping, and may be referred to as RWD (Real World Data) study due to the characteristics of this means. In the examples of the present application, with May 31, 2019 as the cutoff date, the available medical information and genetic information before the cutoff date were extracted.

Specifically, in the study, the assessment item was a pain score expressed by NRS (Numeric Racing Scale; 11-step category variable of 0 to 10) or VAS (continuous variable of 0 to 10) . Three cohorts were set according to the cause of neuropathic pain along with their criteria, and each cohort was further classified into two groups according to the type of the single nucleotide polymorphism in the patient-derived P2X7 receptor gene. Each group was designated as the P2X7 receptor gain-of-function type group (GOF type group) and the P2X7 receptor loss-of-function type group (LOF type group).

Inclusion/exclusion criteria for patients in each of the three cohorts are shown in Tables 10-12.

**[Table 10]**

| Inclusion/Exclusion criteria for patients with postherpetic neuralgia pain (PHN) | | |
|---|---|---|
| Item | Inclusion/Exclusion criteria | Content of criteria |
| Disease code | Inclusion criteria | Any of 1) to 3) must be included: |
| | | 1) Postherpetic neuralgia (including postherpetic trigeminal neuralgia) |
| | | 2) Shingles and neurological symptoms |
| | | 3) Other neurological symptoms after shingles |
| Presence of the pain score to be analyzed | Inclusion criteria | Patient with pain scores (NRS or VAS) observed 7 days or more before the definitive diagnosis of postherpetic neuralgia |
| Gene mutation | Inclusion criteria | One of 1) and 2) must be included: |
| | | 1) having GOF-type haplotypes on both chromosomes (GOF-type patient) |
| | | 2) having a LOF-type haplotype on one or both chromosomes; wherein when only one chromosome has a LOF-type haplotype, the other chromosome has a standard type haplotype (LOF-type patient) |
| Confirmation of diagnosis by a doctor in the biobank | Inclusion criteria | Patient to be diagnosed with PHN by medical judgment based on the confirmation of medical records by a doctor in the biobank. For example, the following points are confirmed: |
| | | 1) a history of shingles can be confirmed |
| | | 2) the site of neurological symptoms is the frequent site of PHN (trigeminal nerve area, abdomen or chest) |
| | | 3) the neurological symptoms are typical (tingling, etc.) |
| Confirmation of causative disease | Inclusion criteria | More than 28 days have passed from shingles to the definitive diagnosis of postherpetic neuralgia. |
| Other cohort diseases | Exclusion criteria | Not suffering from other cohort-related disorders (DPNP or PSP); wherein when pain scores of a patient are recorded after suffering from PHN and before suffering from other cohort disorders, the patient is included in a subject patient. |

**[Table 11]**

| Inclusion/Exclusion criteria for patients with diabetic peripheral neuropathic pain (DPNP) | | |
|---|---|---|
| Item | Inclusion/Exclusion criteria | Content of criteria |
| Disease code | Inclusion criteria | Having a code indicating DPNP or DPNP and other related diseases |
| Presence of the pain score to be analyzed | Inclusion criteria | Patient with pain scores (NRS or VAS) observed 7 days or more before the definitive diagnosis of diabetic neuropathic pain |
| Gene mutation | Inclusion criteria | One of 1) and 2) must be included: |
| | | 1) having GOF-type haplotypes on both chromosomes (GOF-type patient) |
| | | 2) having a LOF-type haplotype on one or both chromosomes; wherein when only one chromosome has a LOF-type haplotype, the other chromosome has a standard type haplotype (LOF-type patient) |
| Confirmation of diagnosis by a doctor in the biobank | Inclusion criteria | Patient to be diagnosed with DPNP by medical judgment based on the confirmation of medical records by a doctor in the biobank. For example, the following points are confirmed: |
| | | 1) the presence of diabetes can be confirmed |
| | | 2) the site of neurological symptoms is the frequent site of DPNP (ends of limbs) |
| | | 3) the neurological symptoms are typical (numbness, etc.) |
| Other cohorts | Exclusion criteria | Not suffering from other cohort-related disorders (PHN or PSP); wherein when pain scores of a patient are recorded after suffering from DPN and before suffering from other cohort disorders, the patient is included in a subject patient. |
| Amputation of limbs | Exclusion criteria | Patient with a history of amputation of the limb before the date of DPNP diagnosis is excluded. |
| | | Patient with a history of amputation of the limb after the date of DPNP diagnosis is excluded when no pain scores are recorded during the period between the date of diagnosis of DPNP and the date of amputation of the limb. |

| | | |
|---|---|---|
| * Since the number of corresponding cases of DPNP patients was much larger than that of other cohorts, about 90 cases in the GOF type group and LOF type group were selected in the process of case extraction for the purpose of extracting the same number of cases as PHN. The selection method is to extract approximately 90 cases from the cases in which "the pain score (A type pain score) is present during the period when no neuropathic pain drug is administered, or the pain score that never affects other pain-related diseases are present" in order from the date of diagnosis specified by the above-mentioned "confirmation of diagnosis by a doctor in the biobank" to the date of the first pain score observed thereafter. | | |

**[Table 12]**

| Inclusion/Exclusion criteria for patients with post-surgical pain (PSP) | | |
|---|---|---|
| Item | Inclusion/Exclusion criteria | Content of criteria |
| Disease code | Inclusion criteria | Having a code indicating post-surgical chronic pain, post-thoracotomy pain, or post-laminectomy pain, or having a code indicating a mastectomy code and a post-surgical neuropathy/neuralgia code |
| pain to be analyzed | Inclusion criteria | Patient with pain scores (NRS or VAS) observed 7 days or more before the definitive diagnosis of post-surgical pain |
| Gene mutation | Inclusion criteria | One of 1) and 2) must be included: |
| | | 1) having GOF-type haplotypes on both chromosomes (GOF-type patient) |
| | | 2) having a LOF-type haplotype on one or both chromosomes; wherein when only one chromosome has a LOF-type haplotype, the other chromosome has a standard type haplotype (LOF-type patient) |
| Confirmation of diagnosis by a doctor | Inclusion criteria | Patient to be diagnosed with PSP by medical judgment based on the confirmation of medical records by a doctor in the biobank. For example, the following points are confirmed: |
| | | 1) the presence of highly invasive surgery to cut peripheral nerves can be confirmed |
| | | 2) the site of neurological symptoms coincides with the site of surgery |
| | | 3) the neurological symptoms are typical (burning, etc.) |
| Other cohorts | Exclusion criteria | Not suffering from other cohort-related disorders (PHN or DPNP) ; wherein when pain scores of a patient are recorded after suffering from DPN and before suffering from other cohort disorders, the patient is included in a subject patient. |

Based on the criteria shown above, some of the extracted patient's medical information (background information, clinical information, etc.) and genetic information (SNPs information for identifying the GOF type group or LOF type group on each of the two chromosomes) are obtained from the biobank.

### (2) Pain score extraction method

The pain score in the recorded medical information cannot always be determined to be pain caused only by the target disease (PHN, DPNP, or PSP). Therefore, for the purpose of analyzing after excluding the influence on the pain score due to other than the target disease as much as possible, the inclusion/exclusion criteria of the pain score were determined as follows.

**[Table 13]**

| Pain score extraction criteria | | |
|---|---|---|
| Item | Inclusion/Exclusion criteria | Content of criteria |
| Recruitment period on the time axis | Inclusion criteria | Adopt pain scores from the date of diagnosis of each cohort disease to 3 years after the date of diagnosis |
| Pain score type | Inclusion criteria | 1) Use pain scores of NRS or VAS that can be assessed from 0 to 10. Namely, five-level scales and non-numerical assessments (Mild/Moderate/Saver, etc.) are not adopted. |
| | | 2) The recall time is "the present time" or "within the past one month". Namely, for example, when pain scores or recall time recalling the "past 3 months" is unknown, it is not adopted. |
| | | 3) When a record is present as the degree of pain, it should be "average" (wherein this rule does not apply to "the present time") . Namely, for example, the "worst pain" score is not adopted. |
| Amputation of limbs | Exclusion criteria | In the case of amputation of limbs, pain scores after amputation surgery are excluded. |
| Score that seems to be acute post-surgical pain (PSP cohort only) | Exclusion criteria | In the PSP cohort, pain scores for 28 days after the causative surgery are excluded. |
| Post-surgery scores for fundamental treatment (PHN cohort only) | Exclusion criteria | In the PHN cohort, when the patient has a surgical code that is considered to be aimed at fundamental treatment, pain scores after the surgical date are excluded. |
| Other complications / previous diseases | Exclusion criteria | For diseases exhibiting pain that are thought to have a significant effect on pain scores, or diseases affecting pain assessment, a period is set for each individual disease code, and pain scores existing within that period are excluded. For example, in the case of an open fracture, pain scores for 90 days from the date of the disease code are excluded, or in the case of Alzheimer's dementia, all pain codes after the date of the disease code are excluded, etc. |
| Other treatments/ surgeries | Exclusion criteria | For each treatment or surgery that is considered to have a significant impact on the pain score, a period is set for each individual treatment code, and pain scores within that period are excluded. |
| | | For example, when arterial blood sampling is performed, pain scores for that day is excluded. |

The pain scores extracted according to the above tables were analyzed, and the average pain score for each patient was calculated. As a result of extraction according to the above tables, patients without a pain score to be analyzed were excluded from the analysis.

The pain scores extracted according to Table 13 were classified into three types, "non-treatment period", "variation period of treatment effect", and "stable period of treatment effect" from the viewpoint of therapeutic property and variability of treatment effect (Fig. 1).

1) Pain score that can be regarded as observed during the non-treatment period (A type pain score)
2) Pain score that can be regarded as observed during the treatment period, wherein the score was observed during the variation period of treatment effect (B-1 type pain score) .
3) Pain score that can be regarded as observed during the treatment period, wherein the score was observed during the stable period of treatment effect (B-2 type pain score).

### (3) Data assessment method

The purpose of this example is to assess the effect of single nucleotide mutations in the human P2X7 receptor gene on neuropathic pain. Thus from the viewpoint of eliminating the effects of other neuropathic pain therapeutic agents as much as possible, the pain score that can be regarded as observed in the non-treatment period, namely, only the A type pain score is assessed of the three types of extracted A-type pain score, B-1 type pain score, and B-2 type pain score. (Assessment I).

In addition, as a result of Assessment I, there were no cases in which PSP could be assessed. From the viewpoint of comparing with the degree of pain alleviation for PSP, B-1 type pain score which is the score of patients in the variation period of treatment effect was excluded, and then A type pain score and B-2 type pain score were assessed of the three types of extracted A type pain score, B-1 type pain score, and B-2 type pain score (Assessment II). Assessment II includes patients in the treatment period for neuropathy pain treatment (B-2 type pain score), but there was almost no difference in the number of patients using neuropathic pain treatment agents between the GOF type group and the LOF type group. Therefore, it can be regarded that there is no difference in the effect of neuropathic pain therapeutic drug treatment. (Strictly speaking, it can be assumed that the pain score of the GOF type group tends to be lower than that of the LOF type group because many more potent opioid analgesics (such as morphine) were prescribed in the GOF type group.)

### 2. Study result

The numbers of patients who met the inclusion criteria of each cohort extracted from the above database are as follows.

**[Table 14]**

| Number of cases in each cohort extracted from the database | | |
|---|---|---|
| Cohort | Number of cases | |
| | Number of cases in the GOF type group | Number of cases in the LOF type group |
| PHN | 42 | 39 |
| DPNP | 44 | 38 |
| PSP | 22 | 23 |

The A-type pain scores of patients who met the inclusion criteria of each extracted cohort are as follows.

**[Table 15]**

| Mean value of A-type pain for each cohort of cases extracted from the database (Assessment I) | | | |
|---|---|---|---|
| Cohort | Mean value of A type pain | | Difference value |
| | Mean value of A type pain in the GOF type group | Mean value of A type pain in the LOF type group | |
| PHN | 5.36 (n=7) | 5.56 (n=3) | -0.20 |
| DPNP | 6.53 (n=8) | 4.76 (n=8) | 1.77 |

| | | | |
|---|---|---|---|
| * PSP has no patients with A type pain score, and thus has no applicable cases. * N represents the number of patients. | | | |

Here, since type A pain is a pain score that can be regarded as being untreated, it is thought that each difference (mean value of type A pain in the GOF type group - mean value of type A pain in the LOF type group) shows how neuropathic pain formed in each cohort or three cohorts is affected by taking a compound that attenuates the human P2X7 receptor function.

Namely, this result is thought to indicate that when a compound that attenuates the human P2X7 receptor function is administered to a DPNP patient, it can act effectively/efficiently in alleviating or treating pain, and theoretically, the pain score will decrease by 1.77.

In addition, the A-type and B-2-type pain scores of patients who met the inclusion criteria of each extracted cohort were as follows.

**[Table 16]**

| Mean value of A-type pain and B-2-type pain in each cohort of cases extracted from the database (Assessment II) | | | |
|---|---|---|---|
| Cohort | Mean value of A type pain and B-2 type pain | | Difference value |
| | Mean value of A-type pain and B-2 type pain in the GOF type group | Mean value of A-type pain and B-2 type pain in the LOF type group | |
| PHN | 5.94 (n=24) | 5.63 (n=20) | 0.31 |
| DPNP | 6.33 (n=26) | 5.62 (n=19) | 0.71 |
| PSP | 6.30 (n=2) | 5.94 (n=4) | 0.36 |

| | | | |
|---|---|---|---|
| * N represents the number of patients. | | | |

This result is thought to indicate that when a compound that attenuates the human P2X7 receptor function is administered to a DPNP patient, it can act effectively/efficiently in alleviating or treating pain, and theoretically, the pain score will decrease by 0.71.

In addition, the result is thought to indicate that when a compound that attenuates the human P2X7 receptor function is administered to a PSP patient, it can act effectively/efficiently in alleviating or treating pain, and theoretically, the pain score will decrease by 0.36.

From the above result, it can be theoretically considered that the compound that attenuates the human P2X7 receptor function may exhibit an excellent efficacy for diabetic peripheral neuropathic pain (DPNP) and postoperative pain (PSP), especially diabetic peripheral neuropathic pain (DPNP) among neuropathic pains.

### [Example 6]

### (Study for assessing the effect of single nucleotide mutations in the human P2X7 receptor gene on the effect of therapeutic drug for neuropathic pain)

### 1. Study method

### (1) Data extraction/inclusion/exclusion method

It was carried out according to "1. Study method", "(1) Case extraction method", and "(2) Pain score extraction method" in Example 5.

### (2) Data assessment method

The purpose of this example is to assess the effect of single nucleotide mutations in the human P2X7 receptor gene on the effects of therapeutic agents for neuropathic pain. Of the three types of extracted A type pain score, B-1 type pain score, and B-2 type pain score, the B-1 type pain score is a pain score that can be regarded as being observed during the treatment period and is a pain score observed during the variation period of treatment effect. Therefore, the difference between the B-2 type pain score and the A type pain score observed in the non-treatment period ([B-2 type pain score] - [A type pain score]) was assessed for the purpose of eliminating the variability of the therapeutic effect.

### 2. Study result

**[Table 17]**

| [Mean value of B-2 type pain score] - [Mean value of A type pain score] in the GOF type group of each cohort extracted from the database | |
|---|---|
| Cohort | GOF type group |
| PHN | -0.19(n=3) |
| DPNP | -0.49(n=8) |
| PHN/DPNP-combined | -0.41(n=11) |

| | |
|---|---|
| * PSP has no patients with A type pain score, and thus has no applicable cases. * N represents the number of patients. | |

When the assessment value is a minus value, it can be considered that the pain score is attenuated, and namely, the pain is improved.

When observing two cohorts, pain improved in the GOF type group. Therefore, the inventors think that it has been theoretically shown that when a therapeutic agent for neuropathic pain is administered to a neuropathic pain patient, a good improving effect on pain can be expected, especially in a patient with a human P2X7 receptor gain-of-function type.

When observing the diabetic peripheral neuropathic pain (DPNP) patient cohort of the two cohorts, it can be seen that the good improving effect on pain in the GOF type group is even more remarkable. Therefore, the inventors think that it has been theoretically shown that when a neuropathic pain therapeutic agent is administered to a patient with diabetic neuropathic pain, an extremely good improving effect on pain can be expected, especially in a patient with a human P2X7 receptor gain-of-fraction type.

All neuropathic pain therapeutic agents used during the treatment period for two cohorts of patients are listed below. These are tricyclic antidepressants (TCA), serotonin-noradrenaline reuptake inhibitors (SNRIs), selective serotonin reuptake inhibitors (SSRIs), calcium channel α2δ subunit binding agents, local anesthetics, opioid analgesics, sodium channel inhibitors, NMDA-type glutamate receptor inhibitors, etc. In addition, since these were used without a large bias in each cohort or each group (for example, there is no difference in the number of patients using the neuropathic pain therapeutic agent), it can be considered that the above-mentioned neuropathic pain therapeutic agents are not particularly limited. Therefore, the agents are not particularly limited as long as they are therapeutic agents that widely alleviate neuropathic pain.

**[Table 18]**

| Neuropathic pain agents used during the treatment period (used in two or more cohorts and listed in descending order of the number of cases used) | | |
|---|---|---|
| NO. | Class | Compound |
| 1 | calcium channel α2δ subunit binding drug | gabapentin |
| 2 | opioid analgesic | hydrocodone |
| 3 | local anesthetic | lidocaine |
| 4 | opioid analgesic | oxycodone |
| 5 | opioid analgesic | fentanyl |
| 6 | opioid analgesic | hydromorphone |
| 7 | opioid analgesic | morphine |
| 8 | serotonin-noradrenaline | duloxetine |
| | reuptake inhibitor (SNRI) | |
| 9 | calcium channel α2δ subunit binding drug | pregabalin |
| 10 | opioid analgesic, etc. | hydrocodone/acetaminophen-combination drug |
| 11 | tricyclic antidepressant (TCA) | amitriptyline |
| 12 | opioid analgesic | tramadol |
| 13 | selective serotonin reuptake inhibitor (SSRI) | citalopram |
| 14 | selective serotonin reuptake inhibitor (SSRI) | sertraline |
| 15 | local anesthetic | benzocaine |
| 16 | opioid analgesic | meperidine (pethidine) |
| 17 | opioid analgesic, etc. | oxycodone/acetaminophen-combination drug |
| 18 | serotonin-noradrenaline reuptake inhibitor (SNRI) | venlafaxine |
| 19 | opioid analgesic | codeine |
| 20 | selective serotonin reuptake inhibitor (SSRI) | escitalopram |
| 21 | sodium channel inhibitor | oxycarbazepine |
| 22 | selective serotonin reuptake inhibitor (SSRI) | fluoxetine |
| 23 | NMDA-type glutamate receptor inhibitor | ketamine |
| 24 | sodium channel inhibitor | lamotrigine |
| 25 | others | capsaicin |
| 26 | tricyclic antidepressant (TCA) | nortriptyline |
| 27 | opioid analgesic | buprenorphine |
| 28 | tricyclic antidepressant (TCA) | imipramine |
| 29 | selective serotonin reuptake inhibitor (SSRI) | paroxetine |
| 30 | tricyclic antidepressant (TCA) | doxepin |
| 31 | serotonin-noradrenaline reuptake inhibitor (SNRI) | milnacipran |
| 32 | opioid analgesic | nalbuphine |
| 33 | opioid analgesic | OxyContin |
| 34 | local anesthetic | cocaine |
| 35 | opioid analgesic | methadone |
| 36 | opioid analgesic | oxymorphone |
| 37 | opioid analgesic | remifentanil |
| 38 | opioid analgesic | sufentanil |
| 39 | opioid analgesic | tapentadol |
| 40 | opioid analgesic, etc. | tramadol/acetaminophen combination drug |

### [Example 7]

### (Study for confirming the central tissue distribution of the P2X7 receptor antagonist compound)

Sufficient exposure of Compound 7 to the cerebrospinal fluid was observed by repeated oral administration of an appropriate amount of Compound 7 to healthy subjects, and the distribution of the P2X7 receptor antagonist compound to the central tissue in humans was confirmed.

### [Industrial Applicability]

The medicament of the present invention can be used for alleviating neuropathic pain and the like. The present invention is extremely useful in the pharmaceutical industry.

## Claims

1. A medicament for alleviating human neuropathic pain in a patient, which comprises a compound that attenuates the function of the P2X7 receptor as an active ingredient.

2. The medicament according to claim 1, wherein the compound that attenuates the function of the P2X7 receptor is at least one selected from any of the following compounds:
(5S,8S)-N-(2,4-dichloro-6-fluorobenzyl)-5-fluoro-8-hydroxy-8-(hydroxymethyl)-5,6,7,8-tetrahydroquinoline-5-carboxamide;
(5S,8S)-N-(2-chloro-4,6-difluorobenzyl)-5-fluoro-8-hydroxy-8-(hydroxymethyl)-5,6,7,8-tetrahydroquinoline-5-carboxamide;
(5S,8S)-N-(2,4-dichloro-6-(hydroxymethyl)benzyl)-5-fluoro-8-hydroxy-8-(hydroxymethyl)-5,6,7,8-tetrahydroquinoline-5-carboxamide;
(5S,8S)-N-((R)-1-(2-chloro-4-fluorophenyl)ethyl)-5-fluoro-8-hydroxy-8-(hydroxymethyl)-5,6,7,8-tetrahydroquinoline-5-carboxamide;
(5S,8S)-N-((3,5-dichloropyridin-2-yl)methyl)-5-fluoro-8-hydroxy-8-(hydroxymethyl)-5,6,7,8-tetrahydroquinoline-5-carboxamide;
(5S,8S)-N-((R)-1-(2,4-dichlorophenyl)ethyl)-5-fluoro-8-hydroxy-8-(hydroxymethyl)-5,6,7,8-tetrahydroquinoline-5-carboxamide;
(5S,8S)-N-(2-chloro-4-fluorobenzyl)-5-fluoro-8-hydroxy-8-(hydroxymethyl)-5,6,7,8-tetrahydroquinoline-5-carboxamide;
(5S,8S)-N-(2-chloro-3,4-difluorobenzyl)-5-fluoro-8-hydroxy-8-(hydroxymethyl)-5,6,7,8-tetrahydroquinoline-5-carboxamide;
(5S,8S)-N-(2,4-dichloro-3-fluorobenzyl)-5-fluoro-8-hydroxy-8-(hydroxymethyl)-5,6,7,8-tetrahydroquinoline-5-carboxamide; and
(5S,8S)-N-(2,4-dichlorobenzyl)-5-fluoro-8-hydroxy-8-(hydroxymethyl)-5,6,7,8-tetrahydroquinoline-5-carboxamide; and pharmaceutically acceptable salts thereof.

3. A medicament for alleviating human neuropathic pain, which is administered to a patient in which the function of the P2X7 receptor is enhanced.

4. The medicament according to claim 3, wherein said human neuropathic pain is human diabetic peripheral neuropathic pain (DPNP).

5. The medicament according to any one of claims 1-4 wherein the patient is a patient with the P2X7 receptor gain-of-function type or a patient with the P2X7 receptor unchanged function type.

6. The medicament according to any one of claims 1-4 wherein the patient is a patient with the P2X7 receptor gain-of-function type.

7. The medicament according to claim 1, wherein the neuropathic pain is diabetic neuropathic pain.

8. The medicament according to claim 1, wherein the neuropathic pain is postoperative pain.

9. The medicament according to claim 1, 7, or 8 wherein the patient is a patient with the P2X7 receptor gain-of-function type or a patient with the P2X7 receptor unchanged function type.

10. The medicament according to claim 1, 7, or 8 wherein the patient is a patient with the P2X7 receptor gain-of-function type.

11. The medicament according to either claim 1 or 7-10 wherein the compound that attenuates the function of the P2X7 receptor is a compound selected from the group consisting of: (5S,8S)-N-(2-chloro-4-fluorobenzyl)-5-fluoro-8-hydroxy-8-(hydroxymethyl)-5,6,7,8-tetrahydroquinoline-5-carboxamide; (5S,8S)-N-(2,4-dichloro-6-fluorobenzyl)-5-fluoro-8-hydroxy-8-(hydroxymethyl)-5,6,7,8-tetrahydroquinoline-5-carboxamide; and (5S,8S)-N-(2-chloro-4,6-difluorobenzyl)-5-fluoro-8-hydroxy-8-(hydroxymethyl)-5,6,7,8-tetrahydroquinoline-5-carboxamide, or a pharmaceutically acceptable salt thereof.

12. The medicament according to either claim 1 or 7-10 wherein the compound that attenuates the function of the P2X7 receptor is selected from the group consisting of:
(5S,8S)-N-(2-chloro-4-fluorobenzyl)-5-fluoro-8-hydroxy-8-(hydroxymethyl)-5,6,7,8-tetrahydroquinoline-5-carboxamide;
(5S,8S)-N-(2,4-dichloro-6-fluorobenzyl)-5-fluoro-8-hydroxy-8-(hydroxymethyl)-5,6,7,8-tetrahydroquinoline-5-carboxamide; and
(5S,8S)-N-(2-chloro-4,6-difluorobenzyl)-5-fluoro-8-hydroxy-8-(hydroxymethyl)-5,6,7,8-tetrahydroquinoline-5-carboxamide.

13. A method for treating human neuropathic pain in a patient wherein the method comprises administering to the patient a compound that attenuates the function of the P2X7 receptor wherein the neuropathic pain is selected from diabetic peripheral neuropathic pain and postoperative pain.

14. The method according to claim 13 wherein the patient is a patient with the P2X7 receptor gain-of-function type or a patient with the P2X7 receptor unchanged function type.

15. The method according to claim 14 wherein the patient is a patient with the P2X7 receptor gain-of-function type.

16. The method according to any one of claims 13-15 wherein the neuropathic pain is diabetic peripheral neuropathic pain.

17. The method according to any one of claims 13-15 wherein the neuropathic pain is postoperative pain.

18. The method according to any one of claims 13-17 wherein the compound that attenuates the function of the P2X7 receptor is selected from the group consisting of:
(5S,8S)-N-(2,4-dichloro-6-fluorobenzyl)-5-fluoro-8-hydroxy-8-(hydroxymethyl)-5,6,7,8-tetrahydroquinoline-5-carboxamide;
(5S,8S)-N-(2-chloro-4,6-difluorobenzyl)-5-fluoro-8-hydroxy-8-(hydroxymethyl)-5,6,7,8-tetrahydroquinoline-5-carboxamide;
(5S,8S)-N-(2,4-dichloro-6-(hydroxymethyl)benzyl)-5-fluoro-8-hydroxy-8-(hydroxymethyl)-5,6,7,8-tetrahydroquinoline-5-carboxamide;
(5S,8S)-N-((R)-1-(2-chloro-4-fluorophenyl)ethyl)-5-fluoro-8-hydroxy-8-(hydroxymethyl)-5,6,7,8-tetrahydroquinoline-5-carboxamide;
(5S,8S)-N-((3,5-dichloropyridin-2-yl)methyl)-5-fluoro-8-hydroxy-8-(hydroxymethyl)-5,6,7,8-tetrahydroquinoline-5-carboxamide;
(5S,8S)-N-((R)-1-(2,4-dichlorophenyl)ethyl)-5-fluoro-8-hydroxy-8-(hydroxymethyl)-5,6,7,8-tetrahydroquinoline-5-carboxamide;
(5S,8S)-N-(2-chloro-4-fluorobenzyl)-5-fluoro-8-hydroxy-8-(hydroxymethyl)-5,6,7,8-tetrahydroquinoline-5-carboxamide;
(5S,8S)-N-(2-chloro-3,4-difluorobenzyl)-5-fluoro-8-hydroxy-8-(hydroxymethyl)-5,6,7,8-tetrahydroquinoline-5-carboxamide;
(5S,8S)-N-(2,4-dichloro-3-fluorobenzyl)-5-fluoro-8-hydroxy-8-(hydroxymethyl)-5,6,7,8-tetrahydroquinoline-5-carboxamide; and
(5S,8S)-N-(2,4-dichlorobenzyl)-5-fluoro-8-hydroxy-8-(hydroxymethyl)-5,6,7,8-tetrahydroquinoline-5-carboxamide; and pharmaceutically acceptable salts thereof.

19. The method according to any one of claims 13-17 wherein the compound that attenuates the function of the P2X7 receptor is a compound selected from the group consisting of:
(5S,8S)-N-(2-chloro-4-fluorobenzyl)-5-fluoro-8-hydroxy-8-(hydroxymethyl)-5,6,7,8-tetrahydroquinoline-5-carboxamide;
(5S,8S)-N-(2,4-dichloro-6-fluorobenzyl)-5-fluoro-8-hydroxy-8-(hydroxymethyl)-5,6,7,8-tetrahydroquinoline-5-carboxamide; and
(5S,8S)-N-(2-chloro-4,6-difluorobenzyl)-5-fluoro-8-hydroxy-8-(hydroxymethyl)-5,6,7,8-tetrahydroquinoline-5-carboxamide, or a pharmaceutically acceptable salt thereof.

20. The method according to any one of claims 13-17 wherein the compound that attenuates the function of the P2X7 receptor is selected from the group consisting of:
(5S,8S)-N-(2-chloro-4-fluorobenzyl)-5-fluoro-8-hydroxy-8-(hydroxymethyl)-5,6,7,8-tetrahydroquinoline-5-carboxamide;
(5S,8S)-N-(2,4-dichloro-6-fluorobenzyl)-5-fluoro-8-hydroxy-8-(hydroxymethyl)-5,6,7,8-tetrahydroquinoline-5-carboxamide; and
(5S,8S)-N-(2-chloro-4,6-difluorobenzyl)-5-fluoro-8-hydroxy-8-(hydroxymethyl)-5,6,7,8-tetrahydroquinoline-5-carboxamide.

21. The use of a compound that attenuates the function of the P2X7 receptor for the manufacture of a medicament for treating human neuropathic pain in a patient.

22. The use according to claim 21 wherein the neuropathic pain is diabetic peripheral neuropathic pain.

23. The use according to claim 21 wherein the neuropathic pain is postoperative neuropathic pain.

24. The use according to any one of claims 21-23 wherein the patient is a patient with the P2X7 receptor gain-of-function type or a patient with the P2X7 receptor unchanged function type.

25. The use according to any one of claims 21-23 wherein the patient is a patient with the P2X7 receptor gain-of-function type.

26. The use according to any one of claims 21-25 wherein the compound that attenuates the function of the P2X7 receptor is selected from the group consisting of:
(5S,8S)-N-(2,4-dichloro-6-fluorobenzyl)-5-fluoro-8-hydroxy-8-(hydroxymethyl)-5,6,7,8-tetrahydroquinoline-5-carboxamide;
(5S,8S)-N-(2-chloro-4,6-difluorobenzyl)-5-fluoro-8-hydroxy-8-(hydroxymethyl)-5,6,7,8-tetrahydroquinoline-5-carboxamide;
(5S,8S)-N-(2,4-dichloro-6-(hydroxymethyl)benzyl)-5-fluoro-8-hydroxy-8-(hydroxymethyl)-5,6,7,8-tetrahydroquinoline-5-carboxamide;
(5S,8S)-N-((R)-1-(2-chloro-4-fluorophenyl)ethyl)-5-fluoro-8-hydroxy-8-(hydroxymethyl)-5,6,7,8-tetrahydroquinoline-5-carboxamide;
(5S,8S)-N-((3,5-dichloropyridin-2-yl)methyl)-5-fluoro-8-hydroxy-8-(hydroxymethyl)-5,6,7,8-tetrahydroquinoline-5-carboxamide;
(5S,8S)-N-((R)-1-(2,4-dichlorophenyl)ethyl)-5-fluoro-8-hydroxy-8-(hydroxymethyl)-5,6,7,8-tetrahydroquinoline-5-carboxamide;
(5S,8S)-N-(2-chloro-4-fluorobenzyl)-5-fluoro-8-hydroxy-8-(hydroxymethyl)-5,6,7,8-tetrahydroquinoline-5-carboxamide;
(5S,8S)-N-(2-chloro-3,4-difluorobenzyl)-5-fluoro-8-hydroxy-8-(hydroxymethyl)-5,6,7,8-tetrahydroquinoline-5-carboxamide;
(5S,8S)-N-(2,4-dichloro-3-fluorobenzyl)-5-fluoro-8-hydroxy-8-(hydroxymethyl)-5,6,7,8-tetrahydroquinoline-5-carboxamide; and
(5S,8S)-N-(2,4-dichlorobenzyl)-5-fluoro-8-hydroxy-8-(hydroxymethyl)-5,6,7,8-tetrahydroquinoline-5-carboxamide; and pharmaceutically acceptable salts thereof.

27. The use according to any one of claims 21-25 wherein the compound that attenuates the function of the P2X7 receptor is a compound selected from the group consisting of:
(5S,8S)-N-(2-chloro-4-fluorobenzyl)-5-fluoro-8-hydroxy-8-(hydroxymethyl)-5,6,7,8-tetrahydroquinoline-5-carboxamide;
(5S,8S)-N-(2,4-dichloro-6-fluorobenzyl)-5-fluoro-8-hydroxy-8-(hydroxymethyl)-5,6,7,8-tetrahydroquinoline-5-carboxamide; and
(5S,8S)-N-(2-chloro-4,6-difluorobenzyl)-5-fluoro-8-hydroxy-8-(hydroxymethyl)-5,6,7,8-tetrahydroquinoline-5-carboxamide, or a pharmaceutically acceptable salt thereof.

28. The use according to any one of claims 21-25 wherein the compound that attenuates the function of the P2X7 receptor is selected from the group consisting of:
(5S,8S)-N-(2-chloro-4-fluorobenzyl)-5-fluoro-8-hydroxy-8-(hydroxymethyl)-5,6,7,8-tetrahydroquinoline-5-carboxamide,
(5S,8S)-N-(2,4-dichloro-6-fluorobenzyl)-5-fluoro-8-hydroxy-8-(hydroxymethyl)-5,6,7,8-tetrahydroquinoline-5-carboxamide; and
(5S,8S)-N-(2-chloro-4,6-difluorobenzyl)-5-fluoro-8-hydroxy-8-(hydroxymethyl)-5,6,7,8-tetrahydroquinoline-5-carboxamide.
